# FASCICULE DE BREVET EUROPEEN

(11) **EP 2 760 815 B1**
(45) Date de publication et mention de la délivrance du brevet: **24.08.2016**
(21) Numéro de dépôt: 13735354.6
(22) Date de dépôt: 12.07.2013
(51) Int. Cl.: C07C 41/09, C07C 41/42, C07C 45/39, C07C 51/367, C07C 47/58, C07C 47/575, C07C 43/23, C07C 65/21

(54) **PROCÉDÉ DE PRÉPARATION D'ALKOXYHYDROXYBENZALDÉHYDE SENSIBLEMENT EXEMPTE D'ALKYL-ALKOXYHYDROXYBENZALDÉHYDE**
VERFAHREN ZUR HERSTELLUNG VON ALKOXY-HYDROXYBENZALDEHYD WEITGEHEND FREI VON ALKYL-ALKOXY-HYDROXYBENZALDEHYD
METHOD FOR PRODUCING ALKOXY-HYDROXYBENZALDEHYDE THAT IS SUBSTANTIALLY FREE OF ALKYL-ALKOXY-HYDROXYBENZALDEHYDE

(30) Priorité: 26.07.2012 FR 1257280
(43) Date de publication de la demande: 06.08.2014
(73) Titulaire: RHODIA OPERATIONS, 75009 Paris (FR)
(72) Inventeur: GAREL, Laurent, 69003 Lyon (FR); GAYET, Hubert, 69800 Saint Priest (FR)
(74) Mandataire: Ridray, Annabelle
(86) Numéro de dépôt international: PCT/EP2013/064859
(87) Numéro de publication internationale: WO 2014/016145

(56) Documents cités:
- FR-A1- 2 664 266
- HANS-RENÉ BJØRSVIK ET AL: "High Selectivity in the Oxidation of Mandelic Acid Derivatives and in O- Methylation of Protocatechualdehyde: New Processes for Synthesis of Vanillin, i so- Vanillin, and Heliotropin", ORGANIC PROCESS RESEARCH & DEVELOPMENT, vol. 4, no. 6, 1 novembre 2000 (2000-11-01), pages 534-543, XP055052720, ISSN: 1083-6160, DOI: 10.1021/op0000529

## Description

La présente invention concerne la préparation d'alkoxyhydroxybenzaldéhyde (« AHBA ») et en particulier sa préparation d'une manière la plus pure possible.

L'invention concerne plus particulièrement la préparation de AHBA à partir de hydroxyphénol (« HP »).

L'invention concerne encore plus spécifiquement la préparation de vanilline (« VA ») et/ou d'éthylvanilline (« EVA »).

### Etat de la technique

Les alkoxyhydroxybenzaldéhydes (« AHBA ») sont des produits très importants utilisés d'abord comme arômes et parfums puis ensuite comme produits intermédiaires dans de nombreux domaines tels que par exemple, l'agrochimie, la pharmacie, la cosmétique et autres industries.

En particulier, la vanilline et l'éthylvanilline sont des produits essentiellement à vocation alimentaire. Il est donc important de proposer sur le marché des produits de haute pureté chimique et de bonne qualité gustative tout en assurant un coût de commercialisation le moins élevé possible.

Ainsi, leur procédé de synthèse nécessite d'améliorer les réactions chimiques mises en oeuvre et de réaliser des opérations de séparation et de purification efficaces.

Il a été proposé différents procédés pour la synthèse des aldéhydes aromatiques. Les procédés les plus importants sont basés sur la fonctionnalisation d'un composé phénolique de départ, par exemple : phénol, catéchol (appelé aussi pyrocatéchol, ou « PC »), un dérivé du catéchol, gaïacol (ou 2-méthoxyphénol), et/ou guéthol (ou 2-éthoxyphénol). Intervient, généralement, dans ce type de procédé, le composé phénolique sous une forme salifiée, par exemple, sous forme d'un sel de sodium : un groupe formyle est additionné en position para du groupe hydroxyle présent sur le cycle benzénique, selon différentes méthodes.

Pour préparer le gaïacol, certains procédés consistent à choisir comme matière première l'ortho-nitrochlorobenzène (ONCB).

Ces procédés présentent de nombreux inconvénients par rapport au procédé partant du pyrocatéchol : manipulation de composés toxiques CMR (cancérogènes, mutagènes et reprotoxiques), comme l'o-anisidine ; nombreuses étapes ; réactions exothermiques et dangereuses, comme la nitration ou l'hydrolyse d'un sel de diazonium ; génération de nombreux effluents ; formation d'impuretés, comme le 5-chlorogaïacol, qui se retrouvent dans la vanilline sous la forme de 6-chlorovanilline, etc.

Par ailleurs, il existe une voie d'accès à la vanilline totalement différente qui consiste à faire réagir en milieu basique, le gaïacol et l'acide glyoxylique conduisant à l'acide 4-hydroxy-3-méthoxymandélique puis à oxyder le condensat obtenu.

Le fait de synthétiser le gaïacol (GA) ou de guéthol (GE) à partir de catéchol est beaucoup plus direct.

Il existe également des procédés de synthèse du para-méthoxyphénol (PMP) et/ou du paraéthoxyphénol (PEP) à partir d'hydroquinone.

Les composés comme GA, GE, PMP et PEP sont des alkoxyphénols (« AP »).

L'ajout de la fonction aldéhyde sur un alkoxyphénol (« AP ») pour donner AHBA peut être réalisé par une réaction entre AP et l'acide glyoxylique puis oxydation du composé résultant. Cependant AHBA préparé selon cette voie comprend comme impureté en particulier le composé alkyl-alkoxyhydroxybenzaldéhyde (« AAHBA »). Or ce mélange est particulièrement difficile à séparer.

Du fait de cette séparation difficile, le mélange AHBA/AAHBA est séparé par cristallisation, ce qui implique l'utilisation de solvants organiques.

Ce problème se pose plus spécialement lors de la préparation de vanilline (« VA ») ou d'éthylvanilline (« EVA ») à partir de benzène-1,2-diol (catéchol, ou pyrocatéchol, dit « PC » dans la présente invention) car l'impureté méthylée de la vanilline ou éthylée de l'éthylvanilline est très difficile à séparer de VA et/ou EVA.

De manière générale l'utilisation de solvants organiques est peu souhaitée du fait notamment de la toxicité des solvants organiques utilisables, des coûts supplémentaires générés par leur utilisation, ou du traitement du solvant dans un but de recyclage ou régénération.

### Buts de l'invention

Ainsi la présente invention a pour but de résoudre les problèmes mentionnés ci-dessus.

En particulier, la présente invention a pour but principal d'améliorer les procédés industriels existants de préparation d'alkoxyhydroxybenzaldéhydes (« AHBA »), et en particulier de vanilline (« VA ») et/ou d'éthylvanilline (« EVA »), notamment du point de vue de la pureté du AHBA, et en particulier de la vanilline (VA) et/ou de l'éthylvanilline (EVA).

L'invention a pour but en particulier d'obtenir ces composés sensiblement exempts d'impuretés alkylalkoxyhydroxybenzaldéhyde (« AAHBA »), et en particulier de 5-méthyl-vanilline (« MEVA ») et de 5-éthyl-éthylvanilline (« EEVA »), lors de la production d'au moins un composé alkoxyhydroxybenzaldéhyde, et typiquement de la vanilline et/ou de l'éthylvanilline.

L'invention a également pour but d'optimiser de tels procédés industriels, et notamment d'améliorer les rendements en AHBA des procédés existants avec une pureté maximale de AHBA.

L'invention a pour but particulier de fournir un procédé de purification d'un flux de VA et/ou EVA, dans lequel VA et/ou EVA est préparé à partir de GA et/ou GE.

L'invention a encore pour but particulier de fournir un procédé de purification d'un flux de VA et/ou EVA, dans lequel VA et/ou EVA est préparé à partir de PC.

L'invention a notamment pour but de fournir de tels procédés lorsqu'ils comprennent une réaction d'O-alkylation de l'hydroxyphénol en phase vapeur pour former l'alkoxyphénol correspondant.

La présente invention a pour but de fabriquer le composé alkoxyphénol de manière industrielle avec un bon rendement, une faible production d'impuretés et/ou leur élimination efficace, et une conversion de l'hydroxyphénol élevée. Cependant, par exemple pour un procédé permettant de préparer le gaïacol, il se forme également du vératrole, obtenu par une méthylation supplémentaire (successive) du gaïacol.

Ainsi la présente invention a pour but de limiter la présence de l'impureté dialkoxybenzène générée lors de la préparation d'au moins un alkoxyphénol à partir d'au moins un hydroxyphénol. La présente invention a pour but en particulier de limiter la présence de vératrole lors de la synthèse de gaïacol.

### Résumé de l'invention

La présente invention permet de résoudre les problèmes mentionnés ci-dessus.

La présente invention permet de préparer un composé alkoxyhydroxybenzaldéhyde de manière industrielle avec une pureté élevée tout en conservant une synthèse avec de bons rendements, une faible production d'impuretés et/ou leur élimination efficace, et une conversion du dihydroxybenzène élevée, et avantageusement avec le recyclage efficace des composés utilisés.

L'invention concerne un procédé de préparation d'au moins un alkoxyhydroxybenzaldéhyde (« AHBA ») à partir d'au moins un hydroxyphénol (« HP »), ledit procédé comprenant la formation d'au moins un alkoxyphénol (« AP ») et d'alkyl-alkoxyphénol (« AAP ») la séparation (S) de AP de AAP, et la préparation dudit AHBA, par ajout d'une fonction aldéhyde sur le composé AP, ladite séparation (S) étant mise en oeuvre avant l'obtention de AHBA, la séparation étant réalisée par la mise en oeuvre d'une ou plusieurs étapes de distillation de AP et de AAP en présence de HP.

Du fait de leur structure AAHBA et AHBA présentent des caractéristiques physiques proches, dont notamment des températures d'ébullition proches. L'homme du métier sépare donc ces composés typiquement par cristallisation. Il serait très difficile d'envisager une séparation de ces produits par distillation du fait principalement de la proximité des températures d'ébullition.

Les inventeurs ont constaté qu'il était possible de fournir un procédé de purification de AHBA préparé à partir de HP en résolvant les problèmes mentionnés ci-dessus.

La présente invention permet en particulier de limiter la formation de l'impureté alkylée, et plus particulièrement de limiter la présence du composé du type alkyl-alkoxyhydroxybenzaldéhyde lors de la production d'au moins un composé alkoxyhydroxybenzaldéhyde, comme la 5-méthyl-vanilline ou la 5-éthyl-éthylvanilline lors de la production de la vanilline et/ou de l'éthylvanilline.

La présente invention permet également d'éliminer l'impureté dialkoxylée également formée, et en particulier le vératrole lors de la production de vanilline.

La synthèse d'alkoxyphénol à partir d'hydroxyphénol est connue que ce soit en phase vapeur ou en milieu triphasique.

Les inventeurs ont notamment découverts qu'en phase vapeur la formation des impuretés du type alkyl-alkoxyhydroxybenzaldéhyde sont limitées lors de la production d'au moins un composé alkoxyhydroxybenzaldéhyde si une séparation est mise en oeuvre avant l'obtention de l'alkoxyhydroxybenzaldéhyde.

### Description détaillée de l'invention

Le procédé de l'invention s'applique tout particulièrement à la préparation de la vanilline et de l'éthylvanilline mais il convient également pour d'autres aldéhydes aromatiques. Le procédé de l'invention s'applique également à la préparation de gaïacol et/ou de guéthol à partir de pyrocatéchol, mais il convient également pour d'autres alkoxyphénols.

Par "hydroxyphénol", on entend au moins un composé de type benzène, substitué ou non, comprenant deux groupes hydroxyles. L'hydroxyphénol peut être substitué par exemple par un ou plusieurs groupes choisi parmi : hydroxyle, alkyle, alkoxy, halogène, nitro, aryle, les groupements comprenant des atomes de carbones pouvant comprendre 1 ou plusieurs hétéroatomes, notamment choisi parmi O, N, et S. Il répond plus particulièrement à la formule (I) suivante : 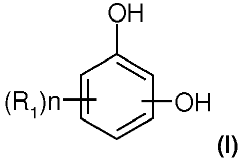 dans ladite formule (I) :
- le ou les groupes R1, identiques ou différents, représentent un atome d'hydrogène, un groupe hydroxyle, un groupe alkyle ou alkoxy ;
- n est un nombre de 0 à 3.

La position respective des deux groupes hydroxyles de la formule (I) n'est donc pas fixée. Elles peuvent être en position ortho, méta, ou para.

On peut citer parmi les composés de formule (I) le pyrocatéchol (« PC » ou ortho-hydroxyphénol), la résorcine (« RS » ou méta-hydroxyphénol) et l'hydroquinone (« HQ » ou para-hydroxyphénol), qui sont préférés pour le procédé selon l'invention.

Dans le cadre de l'invention, on entend par "alkyle", une chaîne hydrocarbonée linéaire ou ramifiée ayant de 1 à 12 atomes de carbone et de préférence de 1 à 4 atomes de carbone. Des exemples préférés de groupes alkyles sont les groupes méthyle, éthyle, propyle, isopropyle et tertio-butyl.

Par "alkoxy", on entend un groupe -O-alkyl dans lequel le terme alkyle a la signification donnée ci-dessus. Des exemples préférés de groupes alkoxy sont les groupes méthoxy et éthoxy.

Par le terme « alkoxyphénol » (AP) l'invention couvre un composé alkoxyphénol seul ou un mélange d'alkoxyphénols. Par le terme « G » l'invention couvre le gaïacol (GA), le guéthol (GE) et l'un quelconque de leurs mélanges.

Par "alkoxyphénol", on entend au moins un composé de type benzène comprenant un groupe hydroxyle et un groupe alkoxy (ou O-alkyl). Il répond plus particulièrement à la formule (II) suivante : 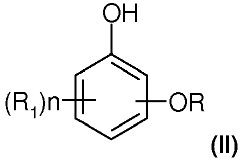 dans ladite formule (II) :
- R représente un groupe alkyle, comprenant de préférence de 1 à 4 atomes de carbone, lié de manière covalente à l'atome d'oxygène ;
- R1, et n sont tels que définis ci-dessus en référence au composé de formule (1).

Les composés alkoxyphénols sont préparés à partir du composé hydroxyphénol correspondant, c'est-à-dire que le groupe R, apporté par un agent d'O-alkylation, remplace un hydrogène d'un des groupes hydroxyle du composé dhydroxyphénol de départ.

On peut citer en particulier le gaïacol, le guéthol, le para-méthoxyphénol (PMP), et le para-éthoxyphénol (PEP).

Par le terme « alkoxyhydroxybenzaldéhyde » (AHBA), l'invention couvre un composé alkoxyhydroxybenzaldéhyde seul ou un mélange d'alkoxyhydroxybenzaldéhydes.
Par "alkoxyhydroxybenzaldéhyde", on entend au moins un composé de type phényle comprenant un groupe hydroxyle, un groupe alkoxy (ou O-alkyl), et un groupe formyle. Il répond plus particulièrement à la formule (III) suivante : 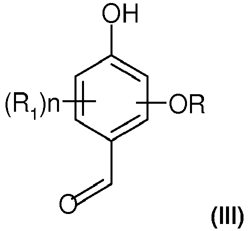 R, R1, et n sont tels que définis ci-dessus en référence au composé de formule (I).

L'alkoxyhydroxybenzaldéhyde mis en oeuvre préférentiellement dans le procédé de l'invention répond à la formule (III) dans laquelle R représente un groupe méthyle ou éthyle et n =0.

Comme exemples plus spécifiques d'alkoxyhydroxybenzaldéhyde, on peut citer notamment :
- la vanilline,
- l'éthylvanilline (3-éthoxy-4-hydroxybenzaldéhyde),
- l'isovanilline (4-méthoxy-3-hydroxybenzaldéhyde),
- l'o-vanilline (3-méthoxy-2-hydroxybenzaldéhyde),
- l'aldéhyde syringique (3,5-diméthoxy-4-hydroxybenzaldéhyde),

Dans la présente invention lorsqu'on fait référence au composé « AHBA », on couvre en particulier « VA et/ou EVA ». Par les termes « VA et/ou EVA » l'invention couvre la vanilline (VA), l'éthylvanilline (EVA), et l'un quelconque de leurs mélanges. Par le terme « V », l'invention fait référence aux composés VA et/ou EVA.

Par le terme « alkyl-alkoxyphénol » (AAP) l'invention couvre un composé alkyl-alkoxyphénol seul ou un mélange d'alkyl-alkoxyphénols. Par « alkyl-alkoxyphénol » on entend un composé du type « alkoxyphénol » selon la formule (II) portant en plus un groupe alkyl sur le noyau benzénique. Par le terme « AG » l'invention couvre un alkyl-gaïacol (AGA), et en particulier le 6-méthylgaïacol (MEGA ou 2-méthoxy-6-méthylphénol), un alkylguéthol (AGE), et en particulier le 6-éthylguéthol (EGE ou 2-éthoxy-6-éthylphénol), et l'un quelconque de leurs mélanges.

Par le terme « alkyl-alkoxyhydroxybenzaldéhyde » (AAHBA) l'invention couvre un composé alkyl-alkoxyhydroxybenzaldéhyde seul ou un mélange d'alkyl-alkoxyhydroxybenzaldéhydes. Par le terme « AV » l'invention couvre la vanilline et/ou l'éthylvanilline comprenant en position 5 du groupe phényle un alkyle, et couvre en particulier la 5-méthyl-vanilline (MEVA ou 4-hydroxy-3-méthoxy-5-méthylbenzaldéhyde) et/ou la 5-éthyl-éthylvanilline (EEVA ou 4-hydroxy-3-éthoxy-5-éthylbenzaldéhyde).

Chaque référence à « AHBA » couvre en particulier « V ». De la même manière chaque référence à l'impureté « AAHBA » couvre en particulier l'impureté « AV », chaque référence à « AP » couvre en particulier « G », et chaque référence à l'impureté « AAHB » couvre en particulier les impuretés « AG », sauf indication contraire.

### Réaction d'O-alkylation

AAHBA est généré principalement lors de la synthèse en phase vapeur du composé AP à partir de HP, synthèse qui comprend par exemple la O-alkylation d'un groupe hydroxyle de HP en présence d'au moins un agent O-alkylant porteur d'au moins un groupe alkyle (appelée ici réaction d'O-alkylation de HP).

La synthèse en phase vapeur d'un alkoxyphénol est connue selon l'art antérieur. On fait notamment référence aux brevets suivants : EP 0 509 927, EP 0 873 786, EP 914 854 et EP 0 933 107. Conformément au procédé de l'invention, on fait réagir au moins un hydroxyphénol avec un agent O-alkylant, en phase vapeur, éventuellement en présence d'un catalyseur, souvent sous forme solide.

On entend par l'expression « phase vapeur » que les différents réactifs sont vaporisés dans les conditions réactionnelles mais le procédé n'exclut pas la présence d'une éventuelle phase liquide très minoritaire. La réaction est réalisée en phase vapeur, éventuellement en présence de solides, comme un ou plusieurs catalyseurs. Selon une variante préférée, le procédé de l'invention est uniquement réalisé avec des réactifs et produits en phase vapeur, seuls le ou les catalyseurs pouvant être solides ou supportés sur un ou plusieurs solides.

Les composés organiques utilisables pour le procédé de la présente invention sont des hydroxyphénols. Particulièrement, les composés organiques utilisables pour le procédé de la présente invention sont choisis parmi les composés aromatiques définis par l'invention, et de préférence le catéchol, l'hydroquinone et le résorcinol.

L'agent O-alkylant peut être choisi parmi composé porteur d'au moins un groupe alkyle, et de préférence d'au moins un groupe méthyle, éthyle, propyle, isopropyle, butyle, isobutyle, butyle tertiaire, amyle, sec-amyle, isoamyle ou d'amyle, chacun ayant de préférence une température d'ébullition de 50 à 200°C, de manière davantage préférée de 60 à 150°C. Parmi les agents O-alkylant utilisabes dans le cadre de la présente invention, on peut notamment citer les alcools ayant de 1 à 12 atomes de carbone, et de préférence en C1-C4. Comme exemples d'agents O-alkylant, on peut citer les alcanols aliphatiques inférieurs ayant de 1 à 5 atomes de carbone, tels que par exemple, le méthanol, l'éthanol, le trifluoroéthanol, le propanol, l'alcool isopropylique, le butanol, le pentanol, l'isopentanol, l'éther monoéthylique d'éthylèneglycol ainsi que les alcools aliphatiques supérieurs ayant au moins 6 et jusqu'à 12 atomes de carbone, tels que par exemple, l'hexanol, l'heptanol, l'isoheptanol, l'octanol, le nonanol, le décanol, le dodécanol. On peut mettre en oeuvre des alcools cycloaliphatiques ayant de 3 à 12 atomes de carbone, tel que, par exemple, le cyclopentanol, le cyclohexanol, le cycloheptanol, le cyclooctanol, le cyclododécanol, le tripropylcyclohexanol, le méthylcyclohexanol et le méthylcycloheptanol. L'agent O-alkylant est de préférence choisi parmi les alcools aliphatiques. Les alcools préférés sont le méthanol, l'éthanol, l'isopropanol et le tertio-butanol, ou l'éthylène glycol. D'autres agents O-alkylant sont envisageables comme les halogénures d'alkyle, dont notamment le chlorure de méthyl ou d'éthyl ; les carbonates d'alkyles comme le carbonate de méthyle ou le carbonate d'éthyle ; ou les phosphate d'alkyle, comme le triméthyle phosphate ou de triéthyle phosphate.

Ainsi la présente invention couvre plus particulièrement les composés AHBA et AP où l'alkoxy est un groupe en C1-C4, et de préférence un groupe méthoxy et/ou un éthoxy.

Conformément au procédé de l'invention, la réaction de O-alkylation est conduite en phase vapeur, en mettant en contact le composé phénolique de formule (I) et l'agent O-alkylant.

La quantité d'agent O-alkylant, de préférence d'alcanol utilisée est égale ou supérieure à la quantité stoechiométrique nécessaire pour éthérifier un ou plusieurs groupements hydroxyle en groupements alkoxy. Généralement, l'agent O-alkylant de préférence l'alcanol est mis en oeuvre en une quantité telle que le rapport entre le nombre de moles de l'agent O-alkylant et le nombre de fonctions hydroxyle du composé phénolique de formule (I) varie entre 0,5 et 500, de préférence entre 2 et 20. Selon une variante, l'hydroxyphénol et l'agent O-alkylant sont de préférence mélangés en un rapport en poids de mélange de 0,5:99,5 à 90:10, de manière encore préférée de 1:99 à 80:20. Avantageusement, ils sont mélangés en un rapport en poids de mélange de 20:80 à 80:20, et encore préférée de 30:70 à 70:30.

Dans la production de l'alkoxyphénol, on peut utiliser un catalyseur de réaction de O-alkylation. Un catalyseur utilisable n'est pas limité à un type spécifique de catalyseur tant que le catalyseur peut accélérer la réaction de O-alkylation en phase vapeur de l'hydroxyphénol par l'agent O-alkylant. Le catalyseur est de préférence choisi parmi les catalyseurs contenant un métal alcalin, par exemple les hydroxydes, carbonates et bicarbonates de métaux alcalins ; les catalyseurs à l'acide p-toluènesulfonique et/ou phosphate d'aluminium ; les catalyseurs Al-B-P ; les catalyseurs de métaux alcalinoterreux Al-B-P ; les catalyseurs Al-B-P-Si ; les catalyseurs Al-B-P-Ca ; les catalyseurs Al-P-Ti-Si ; et les catalyseurs comprenant un composant actif obtenu à partir d'acide borique et d'acide phosphorique et portés sur un véhicule comprenant une alumine inerte.

Pour la production d'alkoxyphénol à partir d'hydroxyphénol, et notamment à partir de pyrocatéchol, les catalyseurs Al-P sont de préférence utilisés. Les catalyseurs Al-P comprennent les catalyseurs de la réaction de O-alkylation Al-P-Ti-Si représentés par AlₐP_{b}Ti_{c}Si_{d}Oₑ (dans laquelle a, b, c, d et e représentent respectivement le nombre d'atome de Al, P, Ti, Si, et O, avec comme rapports atomiques : lorsque a = 1, b = 1,0 à 1,9, c = 0,05 à 0,5, d = 0,05 à 0,2 et e = 4,1 à 7,0) comme décrit dans la publication de brevet japonais non examinée no 4-341 345. Dans la production d'alkoxyphénol à partir de d'hydroxyphénol, le catalyseur Al-P-Ti-Si précité peut être ajouté à un catalyseur contenant 0,004 à 0,015 atome de soufre par atome d'aluminium, pour former un catalyseur composite. On peut également citer les catalyseurs du type AlₐP_{b}Ti_{c}Si_{d}XₑO_{f} où X est Bi, Sb, Bi/Sb, ou S, avec les rapports atomiques a:b =1:1,0 à 1:1,9 ; a:c = 1:0,05 à 1:0,5 ; a:d = 1:0,05 à 1:0,2 ; a:e = 1:0,01 à 1:0,3 quand X représente au moins un atome Sb ou Bi, et a:e = 1:0,004 à 1:0,015 quand X représente S ; et a:f = 1:4,1 à 1:8,4. De tels catalyseurs sont décrits dans le brevet EP 0 873 786.

En ce qui concerne le catalyseur, sa productivité pondérale et horaire (whsv - « weight hourly space velocity ») est de préférence comprise entre 0,1 et 20 h⁻¹, de préférence entre 0,5 et 5 h⁻¹: la productivité pondérale horaire d'un catalyseur étant définie par le rapport pondéral entre le composé phénolique introduit par heure et le catalyseur.

Le catalyseur peut être supporté. Le support du catalyseur est alors de préférence un verre fritté quand le catalyseur est sous forme de poudre et de préférence une grille lorsqu'il est sous forme d'extrudés.

Dans le procédé de la présente invention où la phase vapeur est constituée d'un mélange de plusieurs composants, la phase vapeur est générée par évaporation d'un liquide constitué d'un mélange comprenant au moins un hydroxyphénol et au moins un agent O-alkylant. L'hydroxyphénol et l'agent O-alkylant sont introduits au contact du catalyseur, éventuellement entraînés par un gaz vecteur. Le gaz vecteur est optionnel et est en général un gaz ou un mélange de gaz non réactifs dans les conditions de la réaction. On peut faire appel à des gaz tels que l'azote, l'air, l'argon ou l'hélium. Avantageusement, le rapport volumique entre le gaz vecteur et le composé phénolique varie entre 0 et 10, de préférence entre 0,1 et 2,0.

La pression réactionnelle peut être comprise entre 10⁻² et 50 bars, et de préférence est la pression atmosphérique.

Le liquide constitué d'un mélange de plusieurs composants est de préférence introduit dans un évaporateur à une température d'alimentation d'environ 50 à 300°C, de manière davantage préférée de 80 à 250°C, de manière encore davantage préférée de 100 à 220°C, pour empêcher ou limiter la détérioralon thermique des composants, spécialement ceux ayant une haute température d'ébullition.

Il est possible de vaporiser chacun des réactifs (hydroxyphénol, agent O-alkylant, etc.) dans des chambres différentes, puis d'effectuer le mélange dans une chambre de mélange et d'introduire le flux gazeux résultant sur le catalyseur. Le gaz vecteur peut être introduit en parallèle audit flux gazeux ou bien au niveau de la chambre de mélange.

Une autre variante consiste à préparer une solution comprenant l'hydroxyphénol et l'agent O-alkylant, puis à vaporiser ledit mélange et à l'introduire sur le catalyseur, en parallèle avec le gaz vecteur.

Un autre mode de réalisation pratique de l'invention consiste à faire fondre l'hydroxyphénol en le chauffant jusqu'à sa température de fusion et l'on fait passer au-dessus un flux gazeux comprenant l'agent O-alkylant. Ce flux se sature en hydroxyphénol et il est mis alors en contact avec le catalyseur.

En fin de réaction, on condense l'ensemble des gaz et l'on sépare les réactifs non réagis et les produits obtenus par distillation.

Dans le procédé de la présente invention, par exemple lorsque 100 parties en poids d'hydroxyphénol ayant une température d'ébullition de 150 à 300°C, particulièrement de 180 à 290°C, sous la pression atmosphérique ambiante et 80 à 120 parties en poids d'un agent O-alkylant ayant une température d'ébullition de 50 à 200°C, particulièrement de 60 à 150°C, et de 100 à 200°C,particulièrement de 120 à 180°C, sous la température d'ébullition de l'hydroxyphénol, sont mélangés l'un avec l'autre, le liquide résultant peut être vaporisé puis injecté dans un réacteur de O-alkylation en phase vapeur afin de générer l'alkoxyphénol (AP) d'intérêt. On peut se reporter notamment aux conditions et appareillages préconisés dans le brevet EP 0 914 854 B1 pour plus de détails.

La réaction de O-alkylation en phase vapeur est de préférence effectuée dans un réacteur à une température de 200 à 400°C, de préférence de 220 à 350°C, et encore de préférence de 230 à 300°C, sous une pression atmosphérique ambiante ou une pression accrue, par exemple de 98 à 4 903 kPa (1 à 50 kg/cm²), et de préférence de 147 à 2 942 kPa (1,5 à 30 kg/cm²).

Le temps de contact qui est défini comme le rapport entre le volume apparent de catalyseur et le débit du flux gazeux (qui inclut le gaz vecteur), peut varier largement, et est le plus souvent compris entre 0,5 et 100 secondes. Le temps de contact est choisi de préférence entre 1 et 10 secondes. En ce qui concerne la réalisation pratique de l'invention, on commence à préparer le lit catalytique qui est constitué par le catalyseur posé sur un support (par exemple, verre fritté, grille) ce qui permet la circulation des gaz sans élution du catalyseur. Ensuite, les réactifs sont mis en oeuvre et plusieurs variantes sont possibles.

L'intérêt du procédé, objet de la présente invention est qu'il permet d'obtenir au moins un alkoxyphénol avec une bonne sélectivité de réaction.

Le procédé, objet de la présente invention convient tout à fait bien pour la préparation du gaïacol et/ou du guéthol par réaction de O-alkylation du pyrocatéchol respectivement avec du méthanol et/ou de l'éthanol.

Dans le cas de la O-méthylation du pyrocatéchol, le procédé de l'invention est très intéressant car il permet de moduler la production du gaïacol ou du vératrole selon les besoins du marché en contrôlant la quantité de méthanol et la pression (1 et 20 Bar).

Il en est de même pour la O-méthylation de l'hydroquinone qui permet d'obtenir le p-méthoxyphénol et/ou le p-diméthoxybenzène.

Le procédé de l'invention réalise une O-alkylation sans faire appel aux agents O-alkylant classiques tels que le chlorure de méthylène qui présente une certaine toxicité. De plus il ne conduit pas à des effluents aqueux salins ce qui le rend très intéressant par rapport aux contraintes environnementales et économiques puisqu'il permet de se dispenser d'un traitement des rejets aqueux.

Le produit de réaction délivré à partir du réacteur de O-alkylation contient comme produit cible l'alkoxyphénol, et l'alkyl-alkoxyphénol, comme impureté, l'agent O-alkylant non réagi, l'hydroxyphénol non réagi et des sous-produits. Le flux provenant du réacteur de O-alkylation comprend donc un mélange M comprenant AP et l'impureté AAP.

Les précurseurs des impuretés MEVA et EEVA sont générés en particulier lors de la préparation en phase vapeur de GA et/ou GE à partir de PC par O-alkylation.

Il est donc particulièrement avantageux de séparer AAP du mélange M, afin d'obtenir le composé AP séparé de l'impureté AAP. Cette séparation permet de préparer AHBA sensiblement exempt d'alkyl-alkoxyhydroxybenzaldéhyde (AAHBA). Par « sensiblement exempt » on entend que la quantité d'impureté AAHBA est suffisamment faible pour permettre une utilisation dans l'industrie alimentaire du produit AHBA produit. Cette quantité est de préférence inférieure à 1000 ppm et de préférence inférieure à 500 ppm, et encore de préférence inférieure à 200 ppm par rapport à l'alkoxyhydroxybenzaldéhyde formé.

Selon une variante la présente invention concerne la préparation d'un flux de VA préparée à partir de GA.

Selon une autre variante, la présente invention concerne la préparation d'un flux de EVA préparée à partir de GE.

Selon une autre variante, la présente invention concerne la préparation d'un flux d'un mélange de VA et EVA préparé à partir d'un mélange de GA et GE.

L'invention porte en particulier sur un procédé de purification d'un flux de VA et/ou EVA, notamment obtenu à partir de PC. L'invention porte en outre notamment sur la séparation préalable de l'impureté AAP avant sa transformation en AAHBA, et plus particulièrement de l'impureté MEGA ou EGE avant sa transformation en MEVA ou EEVA. Ainsi l'invention couvre un procédé de purification de VA et/ou EVA obtenu à partir de GA et/ou GE, dans lequel le procédé comprend la séparation (S) de MEVA et/ou EEVA avant l'obtention de VA et/ou EVA.

La séparation (S) selon l'invention permet d'obtenir le composé AHBA, et en particulier VA et/ou EVA, fortement purifié au regard de l'impureté AAHBA (AAHBA inférieur à 100ppm en poids du mélange final comprenant le composé AHBA), et en particulier au regard de l'impureté MEVA ou EGE.

Selon une variante, le procédé de la présente invention comprend la préparation de AHBA par réaction de AP avec l'acide glyoxylique, puis oxydation. Avantageusement, la séparation (S) est réalisée avant l'oxydation du composé résultant de la réaction de AP avec l'acide glyoxylique. De préférence la séparation est réalisée avant la réaction de AP avec l'acide glyoxylique. Ainsi on préfère séparer AAP le plus tôt possible après sa formation lors de la synthèse de AHBA à partir de HP.

Comme indiqué ci-dessus la distillation du mélange VA/MEVA et/ou EVA/EEVA est très difficile. Cela est d'autant plus vrai pour la distillation d'un mélange GA/MEGA et/ou GE/EGE, les points d'ébullition étant encore plus proches.

Il a été découvert de manière fortuite qu'il est possible de séparer GA de MEGA par distillation, et donc de manière plus générale de séparer G de AG.

La séparation (S) est donc avantageusement réalisée par la mise en oeuvre d'une ou plusieurs étapes de distillation. On fait référence dans l'invention à la distillation indépendamment qu'elle comprenne une ou plusieurs étapes de distillation.

En présence de PC, PC influence le mélange de distillation, comprenant G et AG, de sorte qu'il devient possible de collecter AG en pied de colonne de distillation avec PC tandis que G est collecté en tête.

Sauf indication contraire, au sens de l'invention on entend par « colonne (de distillation) » notamment un appareillage de distillation comprenant (i) un dispositif cylindrique formant colonne de distillation, généralement équipé (ii) dans sa partie basse d'un dispositif de chauffage appelé bouilleur, comprenant de préférence également un dispositif de soutirage de flux ou fraction dit de pied de colonne, et comprenant de préférence un dispositif de ré-introduction dans la partie basse de colonne d'au moins une partie du liquide tombant vaporisé, et (iii) dans sa partie haute d'un dispositif de soutirage, éventuellement équipé d'un dispositif de séparation liquide-vapeur appelé condenseur, permettant de soutirer un flux gazeux dit flux ou fraction de tête de colonne, éventuellement condensé partiellement ou totalement sous forme liquide, et comprenant de préférence également un dispositif de ré-introduction dans la partie haute de la colonne d'au moins une partie du liquide condensée. On parle aussi pour la partie basse de colonne de pied de colonne et pour la partie haute de colonne de tête de colonne. Selon une variante préférée, le bouilleur est un bouilleur à film liquide tombant.

Le composé dialkoxylé de HP (DAB ou dialkoxybenzène) est également une impureté du procédé de préparation de V à partir de HP. Ces composés sont typiquement le vératrole (VER ou le 1,2-diméthoxybenzène) et le 1,2-diéthoxybenzène. DAB présente un point d'ébullition plus élevé que AG et DAB devrait empêcher de collecter AG en pied de colonne.

De manière totalement surprenante et contrairement à ce qu'attendaient les inventeurs, lorsque DAB est présent dans le flux à séparer, c'est-à-dire le flux comprenant G, AG et PC, PC influence notamment les volatilités relatives A/G, DAB/G et AG/G.

Plus spécifiquement, les volatilités relatives DAB/G et AG/G peuvent être inversées notamment en tête de colonne par rapport au pied de colonne en présence de PC, ce qui permet de collecter un flux très purifié de G avec une très faible concentration de AG, qui peut être inférieure à 1000ppm, de préférence inférieure à 500ppm, de préférence inférieure à 200ppm, et encore avantageusement inférieure à 100ppm. Si PC n'était pas présent le vératrole présenterait en pied de colonne une volatilité relative par rapport au GA inférieure à la volatilité relative de MEGA/GA et le vératrole se comporterait comme un composé plus « lourd » que le MEGA, ce dernier passant donc plus facilement avec le GA. Or de manière surprenante, en présence de PC les volatilités relatives en pied de colonne sont inversées et MEGA passe préférentiellement, par rapport au vératrole, en pied de colonne, avec le flux contenant PC. L'invention couvre donc notamment un procédé comprenant une distillation dans laquelle la concentration en vératrole collecté avec GA en tête est supérieure à la concentration de MEGA collecté avec GA en tête, PC étant collecté en pied.

Les conditions de distillation sont choisies pour qu'elles imposent un tel inversement des volatilités relatives. On préfère que le flux d'entrée dans la colonne de distillation comprenne au moins 30% en poids de PC par rapport au poids total de flux, et de préférence au moins 35%, et encore de préférence au moins 40% en poids.

Avantageusement, le procédé de l'invention comprend la préparation de VA et/ou EVA, sensiblement exempte de 5-méthylvanilline (« MEVA ») et de 3-éthoxy-5-éthyl-4-hydroxy-benzaldéhyde (« EEVA »), à partir de gaïacol (GA) et/ou de guéthol (GE), ledit procédé comprenant l'obtention d'un mélange (M) comprenant GA et/ou GE en présence de l'impureté 6-méthylgaïacol (« MEGA ») et/ou 6-éthylguéthol (2-éthoxy-6-éthyl-phénol dit « EGE »), ledit procédé comprenant la séparation (S) de GA et/ou GE de MEGA et/ou EGE, ladite séparation (S) étant mise en oeuvre avant l'obtention de VA et/ou EVA.

Selon une variante de l'invention, le composé AHBA est préparé par ajout d'une fonction aldéhyde au composé AP par condensation avec l'acide glyoxylique puis oxydation du composé résultant, et la séparation (S) est réalisée avant l'oxydation en composé AHBA.

Selon une autre variante de l'invention, la séparation est réalisée avant la réaction de AP avec l'acide glyoxylique.

Ces différentes variantes peuvent être combinées entre elles.

Avantageusement, la séparation est réalisée par la mise en oeuvre d'une ou plusieurs étapes de distillation.

Selon le procédé de l'invention, la distillation peut être réalisée en continu ou en discontinu, mais de préférence en continu. Selon l'invention, la colonne de distillation peut être adaptée au degré de pureté souhaité, et elle peut être constituée de matériaux adaptés aux conditions acides du mélange à distiller.

De préférence, le procédé de l'invention comprend l'introduction d'un flux ou fraction (E1) du mélange M comprenant GA et/ou GE, désigné(s) par « G », et MEGA et/ou EGE, désigné(s) par « AG », en présence de pyrocatéchol (PC) dans une colonne de distillation (E) et la récupération de G essentiellement en tête de colonne de distillation dans un flux ou fraction (E2) et de PC et AG essentiellement en pied de colonne de distillation dans un flux ou fraction (E3).

Selon une variante, ledit mélange M comprend également un dialkoxybenzaldéhyde (DAB), DAB étant en particulier le vératrole (VER), et le composé DAB est récupéré essentiellement en pied de colonne de distillation dans le flux ou fraction (E3).

Selon un mode de réalisation, PC représente de 25 à 75 %, et de préférence de 35 à 65%, et encore de préférence de 40 à 45 %, en poids du poids total du flux ou fraction (E1).

Selon un mode de réalisation, G représente de 25 à 75 %, et de préférence de 35 à 65%, et encore de préférence de 45 à 50 %, en poids du poids total du flux ou fraction (E1).

Selon un mode de réalisation, G représente plus de 90 %, et de préférence plus de 95 %, et encore de préférence plus de 99%, en poids du poids total du flux ou fraction (E2), et de préférence caractérisé en ce que AG représente moins de 1 %, et de préférence moins de 0,5 % et encore de préférence moins de 0,2 %, en poids par rapport au poids total du flux ou fraction (E2).

Avantageusement, le flux ou fraction (E2) comprend de 90 à 100%, et de préférence de 95 à 100 %, et encore de préférence de 99 à 100 %, de G, de 0 à 1% et de préférence moins de 0,5 % et encore de préférence moins de 0,2 %, de AG ; et de 0 à 1% et de préférence moins de 0,5 % et encore de préférence moins de 0,2 %, de VER, les pourcentages étant exprimés en poids par rapport au poids total du flux ou fraction (E2).

Avantageusement, PC représente plus de 60 %, et de préférence plus de 70 %, et encore de préférence plus de 75%, en poids du poids total du flux ou fraction (E3), et de préférence AG représente moins de 8 %, et de préférence moins de 5 % et encore de préférence moins de 3 %, en poids par rapport au poids total du flux ou fraction (E3).

De préférence, le flux ou fraction (E3) comprend de 60 à 100%, et de préférence de 70 à 100 %, et encore de préférence de 75 à 100 %, de PC, de 0 à 8% et de préférence moins de 5 % et encore de préférence moins de 3 %, de AG ; et de 0 à 10% et de préférence moins de 8 % et encore de préférence moins de 5 %, de VER, les pourcentages étant exprimés en poids par rapport au poids total du flux ou fraction (E3).

On décrit encore un procédé de séparation de GA et/ou GE d'un mélange (M) comprenant GA et/ou GE et une impureté 6-méthylgaïacol (« MEGA ») et/ou 6-éthylguéthol (2-éthoxy-6-éthyl-phénol dit « EGE »), dans lequel le mélange M est introduit dans une colonne de distillation en présence de pyrocatéchol (PC) et la récupération d'un flux ou fraction comprenant GA et/ou GE en tête de colonne de distillation et d'un flux ou fraction comprenant de PC, MEGA et/ou EGE en pied de colonne de distillation.

On décrit également un procédé de synthèse d'au moins un alkoxyphénol dit « AP » en phase vapeur, comprenant la formation d'au moins un AP à partir d'une réaction, dite de O-alkylation, en phase vapeur entre au moins un hydroxyphénol dit «HP», et au moins un agent O-alkylant, ladite réaction formant également au moins une impureté alkyl-alkoxyphénol dite « AAP », ladite réaction formant ainsi un mélange M comprenant AP, AAP et HP, ledit procédé comprenant l'introduction du mélange M dans au moins une colonne de distillation et la récupération d'un flux ou fraction comprenant AP en tête de colonne de distillation et d'un flux ou fraction comprenant HP et AAP en pied de colonne de distillation, ledit procédé comprenant éventuellement un recyclage du flux ou fraction comprenant AP en aval de la réaction de O-alkylation et/ou un recyclage du flux ou fraction comprenant HP en amont de la réaction de O-alkylation.

Avantageusement, le procédé comprend la formation d'au moins du gaïacol (GA) et/ou de guéthol (GE) à partir d'une réaction, dite de O-alkylation, en phase vapeur entre le pyrocatéchol dit «PC», et au moins un agent O-alkylant, ladite réaction formant également au moins une impureté 6-méthylgaïacol (« MEGA ») et/ou 6-éthylguéthol (2-éthoxy-6-éthyl-phénol dit « EGE », ladite réaction formant ainsi un mélange M comprenant GA et/ou GE, MEGA et/ou EGE, et PC, ledit procédé comprenant l'introduction du mélange M dans au moins une colonne de distillation et la récupération d'un flux ou fraction comprenant GA et/ou GE en tête de colonne de distillation et d'un flux ou fraction comprenant PC et MEGA et/ou EGE en pied de colonne de distillation, ledit procédé comprenant éventuellement un recyclage du flux ou fraction comprenant GA et/ou GE en aval de la réaction de O-alkylation et/ou un recyclage du flux ou fraction comprenant PC en amont de la réaction de O-alkylation.

De préférence, la distillation dans la colonne (E) est réalisée à une température comprise entre 80 et 200°C. Plus précisément on préfère maintenir une température comprise entre 120 et 180°C en pied de colonne. On préfère maintenir une température de 80 à 120°C en tête de colonne. Dans le cadre de l'invention, et sauf mention contraire, la température en pied de colonne de distillation correspond à la température dans le bouilleur.

Selon un mode de réalisation, la distillation dans la colonne (E) est réalisée à pression atmosphérique. De préférence, la distillation est réalisée sous vide partiel, et de préférence, notamment à une pression allant de 1 à 300 mbar (0,1 à 30 kPa), de préférence de 15 à 100 mbar (1,5 à 10 kPa).

Selon un mode de réalisation, la colonne (E) comprend de 12 à 70 plateaux théoriques, et de préférence entre 20 et 60 plateaux théoriques. On préfère réaliser une alimentation du flux ou fraction (E1) au niveau d'un plateau théorique situé entre 5% et 70%, et de préférence entre 10 % et 60 % du nombre de plateaux théoriques total de la colonne de distillation (E). Avantageusement introduction du flux d'alimentation (E1) est réalisée dans la partie centrale de la colonne entre les plateaux théoriques n°12 et n°40, et de préférence entre les plateaux théoriques n° 15 et n° 30.

Selon un mode de réalisation, la distillation dans la colonne (E) est réalisée à taux de reflux est compris entre 1 et 10, et de préférence entre 2 et 8.

L'invention couvre en outre un procédé de purification de GA et/ou GE obtenu à partir de PC. En particulier, ce procédé comprend une étape de séparation (S) comme décrit ci-dessus, selon l'une quelconque des variantes des modes de réalisation, permettant de séparer l'impureté AG de G obtenu.

Le procédé selon l'invention permet également le recyclage de PC, en particulier suite à l'étape de séparation (S) par distillation.

Par ailleurs, suite à la séparation de l'impureté AAP du mélange M, le flux ou fraction (E3) comprenant PC soutiré en pied de colonne (E) comprend alors AAP comme impureté : PC et AAP sont donc en mélange dans le flux ou fraction (E3) en sortie de pied de colonne de distillation (E). Le recyclage de PC selon l'invention peut donc comprendre avantageusement une distillation de PC afin de séparer PC de l'impureté AAP. L'invention cherche par ailleurs à éliminer l'impureté AG contenue dans un flux ou fraction contenant PC car l'impureté AG peut donner lieu à l'obtention de différents dérivés toxiques.

Cette séparation est difficile. L'invention concerne donc un procédé de séparation de AG d'un mélange contenant AG et PC.

Il est notamment particulièrement difficile d'obtenir PC en présence de moins de 4% et de préférence de moins de 1 %, de AG en poids par rapport au poids total de PC.

Pour obtenir PC avec une telle pureté au regard de AG (typiquement MEGA), il a été constaté qu'il était nécessaire de réaliser une distillation supplémentaire dans une colonne (F) du flux ou fraction du pied de colonne de distillation (E). En général ce flux ou fraction (E3) de sortie de colonne de distillation (E) comprend également l'impureté DAB (VER et/ou le 1,2-diéthoxybenzène), difficile à séparer également de PC. L'invention vise à fournir un flux ou fraction (F6) contenant PC avec moins de 10 %, et de préférence moins de 8 % en poids total des impuretés AG et DAB par rapport au poids total de PC.

A cet effet, on préconise de réaliser avantageusement au moins une distillation avec un soutirage latéral du flux ou fraction (F6) contenant la plus grande quantité de PC. Selon un mode de réalisation avantageux, le procédé comprend donc une distillation supplémentaire dans une colonne (F) du flux ou fraction (E3) issu du pied de colonne de distillation (E) en réalisant un soutirage latéral d'un flux ou fraction (F6) de la colonne (F) pour soutirer un flux ou fraction enrichie en PC. L'absence de soutirage latéral se traduirait notamment par une quantité non désirable de DAB et/ou de AG dans le flux ou fraction soutiré contenant majoritairement PC. Cette mise en oeuvre permet de soutirer un flux ou fraction (F4) en tête de colonne (F) comprenant également majoritairement PC, et des résidus de G, DAB et AG. Le flux ou fraction (F5) de pied de colonne (F) comprend essentiellement des goudrons. Le flux ou fraction (F4) peut être à nouveau fractionné dans une colonne de distillation (F2) pour collecter un flux ou fraction (F7) en pied de colonne (F2), contenant PC et où G, DAB et AG sont en quantité en poids au moins deux fois moins importante que dans le flux (F4), et collecter un flux ou fraction (F8) en tête de colonne (F2) comprenant majoritairement G, DAB et AG. Avantageusement le flux ou fraction (F7) contenant PC est mélangé avec le flux ou fraction (F6) pour avantageusement les recycler vers la réaction d'alkylation de PC. Avantageusement le flux ou fraction en tête de colonne (F2) peut être mélangé avec le flux ou fraction (E1) entrant dans la colonne de distillation (E) pour en effectuer son recyclage.

De préférence, la distillation dans la colonne (F) est réalisée à une température comprise entre 120 et 250°C. Plus précisément on préfère maintenir une température comprise entre 180 et 220°C en pied de colonne. On préfère maintenir une température de 120 à 180°C en tête de colonne.

Selon un mode de réalisation, la distillation dans la colonne (F) est réalisée à pression atmosphérique. De préférence, la distillation est réalisée sous vide partiel, et de préférence, notamment à une pression allant de 1 à 300 mbar (0,1 à 30 kPa), de préférence de 15 à 100 mbar (1,5 à 10 kPa).

Selon un mode de réalisation, la colonne (F) comprend de 8 à 50 plateaux théoriques, et de préférence entre 10 et 40 plateaux théoriques. On préfère réaliser une alimentation du flux ou fraction (F1) au niveau d'un plateau théorique situé entre 5% et 70%, et de préférence entre 10 % et 60 % du nombre de plateaux théoriques total de la colonne de distillation (F). Avantageusement introduction du flux d'alimentation (F1) est réalisée dans la partie centrale de la colonne entre les plateaux théoriques n°2 et n°20, et de préférence entre les plateaux théoriques n°5 etn°20.

Selon un mode de réalisation, la distillation dans la colonne (F) est réalisée à taux de reflux compris entre 1 et 10, et de préférence entre 1 et 8.

De préférence, la distillation dans la colonne (F2) est réalisée à une température comprise entre 80 et 200°C. Plus précisément on préfère maintenir une température comprise entre 120 et 180°C en pied de colonne. On préfère maintenir une température de 80 à 140°C en tête de colonne.

Selon un mode de réalisation, la distillation dans la colonne (F2) est réalisée à pression atmosphérique. De préférence, la distillation est réalisée sous vide partiel, et de préférence, notamment à une pression allant de 1 à 300 mbar (0,1 à 30 kPa), de préférence de 15 à 100 mbar (1,5 à 10 kPa).

Selon un mode de réalisation, la colonne (F2) comprend de 1 à 20 plateaux théoriques, et de préférence entre 2 et 10 plateaux théoriques. On préfère réaliser une alimentation du flux ou fraction (F4) au niveau d'un plateau théorique situé entre 5% et 70%, et de préférence entre 10 % et 60 % du nombre de plateaux théoriques total de la colonne de distillation (F2). Avantageusement l'introduction du flux d'alimentation (F4) est réalisée dans la partie centrale de la colonne entre les plateaux théoriques n° 1 et n° 15, et de préférence entre les plateaux théoriques n° 2 et n° 10.

Selon un mode de réalisation, la distillation dans la colonne (F2) est réalisée à taux de reflux compris entre 1 et 10, et de préférence entre 2 et 8.

Selon un mode de réalisation particulier, la distillation comprend au préalable une séparation, de préférence par distillation dans une colonne (A), des composés les plus légers de ceux les plus lourds pour soutirer en tête de colonne (A) un flux ou fraction (A1) contenant les composés légers et en pied de colonne (A) un flux ou fraction (A2) contenant les composés lourds dont AP. Cette séparation permet notamment d'éliminer l'agent O-alkylant n'ayant pas réagi lors de la réaction avec HP, et notamment PC. Selon une variante le flux ou fraction (A2) est utilisé comme flux d'entrée de colonne de distillation (E), dénommée ci-dessus flux ou fraction (E1).

De préférence, la distillation dans la colonne (A) est réalisée à une température comprise entre 80 et 250°C. Plus précisément on préfère maintenir une température comprise entre 130 et 220°C en pied de colonne. On préfère maintenir une température de 80 à 150°C en tête de colonne.

Selon un mode de réalisation, la distillation dans la colonne (A) est réalisée à pression atmosphérique. De préférence, la distillation est réalisée sous vide partiel, et de préférence, notamment à une pression allant de 1 à 300 mbar (0,1 à 30 kPa), de préférence de 15 à 100 mbar (1,5 à 10 kPa).

Selon un mode de réalisation, la colonne (A) comprend de 8 à 50 plateaux théoriques, et de préférence entre 10 et 40 plateaux théoriques. On préfère réaliser une alimentation du flux ou fraction (A1) au niveau d'un plateau théorique situé entre 5% et 70%, et de préférence entre 10 % et 60 % du nombre de plateaux théoriques total de la colonne de distillation (A). Avantageusement l'introduction du flux d'alimentation (A1) est réalisée dans la partie centrale de la colonne entre les plateaux théoriques n°2 et n°20, et de préférence entre les plateaux théoriques n°5 et n°20.

Selon un mode de réalisation, la distillation dans la colonne (A) est réalisée à taux de reflux compris entre 0,01 et 10, et de préférence entre 0,1 et 5.

Les colonnes peuvent être ensuite dimensionnées selon les connaissances de l'homme du métier.

L'invention couvre également la préparation d'au moins un alkoxyhydroxybenzaldéhyde à partir d'hydroxyphénol.

### Synthèse d'alkoxyhydroxybenzaldéhyde

Le procédé selon l'invention permet de préparer un alkoxyhydroxybenzaldéhyde par ajout d'une fonction aldéhyde sur le composé alkoxyphénol précité.

Le procédé selon l'invention permet de préparer en particulier la vanilline et/ou l'éthylvanilline à partir du gaïacol et/ou du guéthol, notamment du gaïacol et/ou du guéthol purifié(s) selon le procédé de l'invention. Le mélange gaïacol/guéthol est obtenu selon toutes proportions. Le ratio Ga/Ge dépend des conditions de O-alkylation. Ce ratio massique Ga/Ge peut par exemple varier de 0,1/100 à 100/1. /Le ratio massique vanilline/éthylvanilline varie de préférence de 80/20 à 40/60, et encore de préférence de 80/20 à 45/55. Selon une variante, le ratio molaire vanilline/éthylvanilline est au moins égal à 2. De préférence la proportion de vanilline en poids est comprise entre 65 et 72%, et de préférence entre 60 et 70 %, et la proportion d'éthylvanilline est comprise entre 28 et 35% et de préférence entre 30 et 33 % en poids.

Il est fait référence aux composés alkoxyphénol, gaïacol, guéthol, et aux alkoxyhydroxybenzaldéhydes correspondants indépendamment de leur forme salifiée ou non. Lorsqu'on fait explicitement référence au sel, cela permet de décrire un détail particulier sur cet aspect. L'homme du métier est à même de savoir lorsqu'il est en présence du composé salifié ou non, notamment au vu de la littérature citée et de ses connaissances générales.

La fonction aldéhyde est apportée avantageusement selon deux réactions principales, à savoir une condensation du composé alkoxyphénol avec l'acide glyoxylique et l'oxydation du composé résultant de la réaction de condensation.

Selon un mode de réalisation préféré, le procédé de l'invention comprend en outre les étapes suivantes :
- condensation du composé alkoxyphénol avec l'acide glyoxylique pour obtenir le composé mandélique correspondant;
- séparation du composé alkoxyphénol de la solution contenant le composé mandélique, avec de préférence le recyclage du composé alkoxyphénol;
- oxydation du composé mandélique pour obtenir l'alkoxyhydroxybenzaldéhyde;
- séparation de l'alkoxyhydroxybenzaldéhyde par extraction avec un solvant organique, puis séparation de l'alkoxyhydroxybenzaldéhyde du solvant organique avec de préférence le recyclage du solvant organique d'extraction de l'alkoxyhydroxybenzaldéhyde; et
- récupération de l'alkoxyhydroxybenzaldéhyde éventuellement sous forme solide.

Lorsque l'on mentionne sans précision « mandélique », « vanilline », ou « éthylvanilliine », on vise le composé para-mandélique, la para-vanilline, ou l'éthyl-para-vanilline respectivement, sauf indication contraire.

Les composés para-mandéliques (formule (IV)), en particulier la préparation de la para-vanilline, ont les formules suivantes :

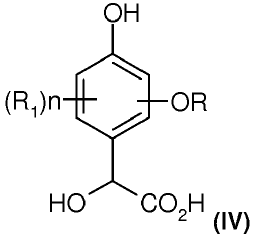

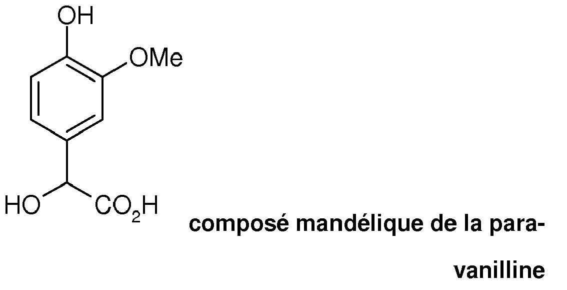

De préférence les étapes et leur enchaînement sont réalisés de manière continue, à l'exception de quelques étapes comme lors de la séparation des composés.

Les réactions de condensation des alkoxyphénols, et en particulier du gaïacol, avec l'acide glyoxylique sont décrites dans la littérature. On citera tout particulièrement les brevets EP 0 578 550, WO 99/65853, et WO 09077383 auxquels on fait référence pour la préparation des composés de l'invention.

L'enchaînement des étapes précédentes peut être réalisé selon les réactions suivantes selon l'exemple du gaïacol :
Condensation du gaïacol et de l'Acide glyoxylique pour former l'acide para-mandélique :

   gaïacol + acide glyoxylique + NaOH → gaïacolate + mandélate (phénate) + H₂O
Acidification pour récupérer le gaïacol non réagi :

gaïacolate + mandélate (phénate) + H₂SO₄ → gaïacol + mandélate + Na₂SO₄

Oxydation de l'acide para-mandélique pour former la para-Vanilline :

mandélate + N_{A}OH + O₂ → vanillate + Na₂CO₃ + H₂O

Acidification pour former la vanilline :

vanillate + Na₂CO₃ + H₂SO₄ → VA + CO₂ + Na₂SO₄

Lors de la séparation du mélange, que ce soit de l'alkoxyphénol, ou de l'alkoxyhydroxybenzaldéhyde ayant conduit à leur formation, on met de préférence en oeuvre un recyclage, appelé également boucle. Ainsi on fait référence dans la présente invention à la « boucle de recyclage 1 » lorsque l'on parle du recyclage de l'alkoxyphénol en excès lors de la réaction de condensation, et de « boucle de recyclage 2 » lorsque l'on fait référence au recyclage du solvant d'extraction de l'alkoxyhydroxybenzaldéhyde, lors des étapes de séparation.

L'alkoxyhydroxybenzaldéhyde une fois séparé peut-être traité pour l'obtenir sous forme purifiée solide, on parle dans la présente invention de « chaine solide ». Ce traitement peut typiquement mettre en oeuvre des étapes de cristallisation, essorage, et séchage, ou écaillage. Parmi ces étapes, certaines, comme l'essorage peuvent ne pas être effectuées de manière continue. Ainsi, de préférence le procédé est continu, à l'exclusion de l'essorage.

La vanilline et/ou l'éthylvanilline ainsi obtenue peut en particulier être utilisée comme arôme dans l'industrie comme par exemple dans l'industrie alimentaire, pharmaceutique ou cosmétique, notamment par exemple pour fabriquer des parfums.

La vanilline et/ou l'éthylvanilline peut également servir d'intermédiaire de synthèse par exemple pour la préparation d'aldéhyde vératrique (3,4 diméthoxybenzaldéhyde) et/ou 3,4-diéthoxybenzaldéhyde.

Dans ce cadre, si le procédé concerne la préparation simultanée de la vanilline et de l'éthylvanilline, on peut soit synthétiser selon un même flux la vanilline et de l'éthylvanilline, à partir d'un flux comprenant du gaïacol et du guéthol, eux-mêmes préparés à partir de catéchol par réaction simultanée avec un agent O-méthylant et un agent O-éthylant ; soit synthétiser séparément du gaïacol et du guéthol puis les mélanger avant l'étape de condensation pour synthétiser selon un même flux la vanilline et de l'éthylvanilline ; soit synthétiser séparément les composés mandéliques par réactions séparées du gaïacol et du guéthol avec l'acide glyoxylique puis mélanger les composés mandéliques correspondants avant l'étape d'oxydation ; soit synthétiser séparément la vanilline et l'éthylvanilline puis les mélanger, par exemple avant la cristallisation.

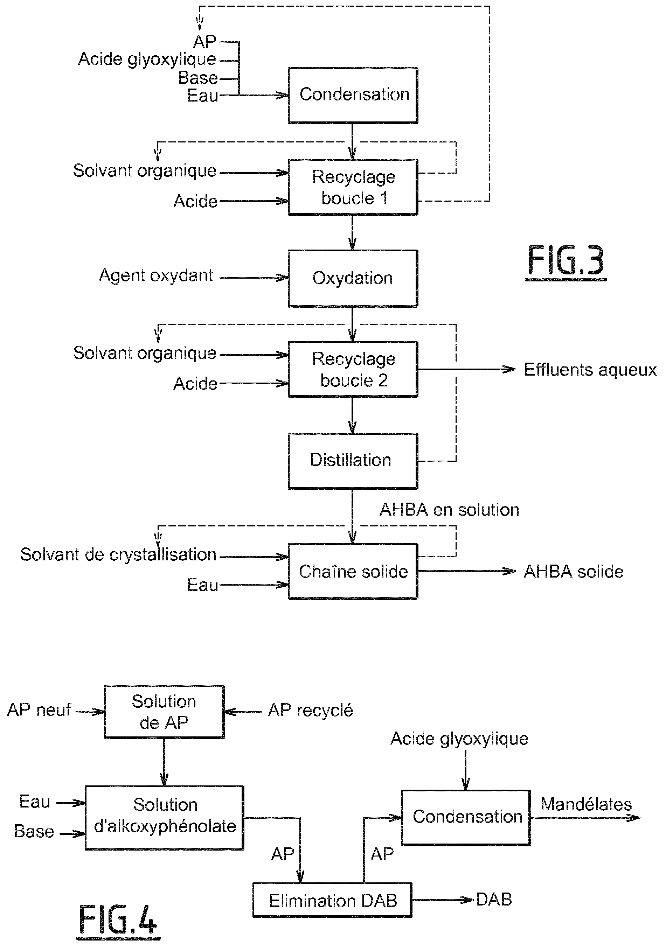

Ainsi on peut représenter un schéma du procédé selon la figure 3 qui comprend avantageusement les étapes suivantes, après l'obtention de AP selon l'invention :
- condensation du composé alkoxyphénol (AP) avec l'acide glyoxylique pour obtenir le composé mandélique correspondant, après ajout d'eau, d'acide glyoxylique et d'une base ;
- séparation du composé alkoxyphénol de la solution contenant le composé mandélique correspondant, après ajout d'un solvant organique et d'un acide, avec de préférence le recyclage du composé alkoxyphénol;
- oxydation du composé mandélique par réaction avec un agent oxydant introduit pour obtenir l'alkoxyhydroxybenzaldéhyde correspondant ;
- séparation de l'alkoxyhydroxybenzaldéhyde par extraction après ajout d'un solvant organique et d'un acide,
- séparation de l'alkoxyhydroxybenzaldéhyde du solvant organique par distillation, avec de préférence le recyclage du solvant organique; et
- récupération de l'alkoxyhydroxybenzaldéhyde (AHBA), éventuellement sous forme solide.

### Réaction de condensation

Selon une variante avantageuse, l'alkoxyphénol est mis à réagir avec l'acide glyoxylique en présence d'une base minérale ou d'une base organique, et de préférence NaOH ou KOH, et encore de préférence en présence de soude (NaOH), ou l'hydroxyde d'ammonium. Selon cette variante on obtient un mélange d'alkylphénolate et de dérivé du type mandélate.

La réaction de condensation entre l'alkoxyphénol (sous forme de phénolate) avec l'acide glyoxylique permet la synthèse de l'acide para-mandélique correspondant.
La condensation peut être réalisée dans des réacteurs agités en cascade. Selon une variante, la réaction est réalisée dans un réacteur à écoulement piston, éventuellement comprenant un échangeur thermique. Un tel mode de réalisation est par exemple décrit pour le gaïacol dans la demande WO 09/077383. La réaction de condensation entre l'alkoxyphénol et l'acide glyoxylique peut être réalisée dans l'eau, en présence d'un métal alcalin, ladite réaction étant conduite en réacteur à écoulement piston. Elle peut être mise en oeuvre dans un réacteur tubulaire.

La réaction de condensation peut être avantageusement catalysée par un hydroxyde d'ammonium quaternaire, comme par exemple selon la réaction décrite dans la demande de brevet EP 0 578 550.
La réaction de condensation peut être avantageusement réalisée en présence d'un acide dicarboxylique, comme par exemple selon la réaction décrite dans la demande de brevet WO 99/65853.

L'alkoxyphénol, étant habituellement en excès par rapport à l'acide glyoxylique, la fraction d'alkoxyphénol n'ayant pas réagi est avantageusement récupérée de la boucle de recyclage 1. Cet excès diminue la probabilité de former des composés du type acide dimandélique.(c'est-à-dire des composés issus de la condensation de deux molécules d'acides glyoxylique sur une molécule de gaïacol).

Avantageusement, dans un 1^{er} temps, l'alkoxyphénol et la soude réagissent pour former l'alkoxyphénolate de soude. Par exemple pour le gaïacol :

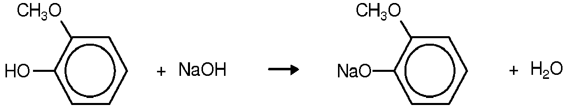

Puis l'alkoxyphénolate réagit avec l'acide glyoxylique pour former le para-mandélate correspondant :

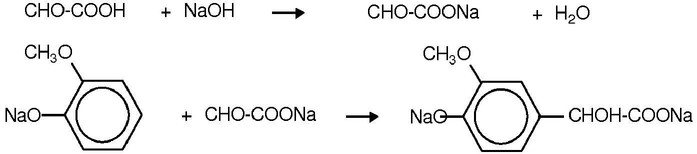

Ces deux réactions de préparation du glyoxylate et du gaïacolate peuvent être mises en oeuvre selon deux étapes séparées. Alternativement l'acide glyoxylique mis en contact directement avec le gaïacolate en présence de la base.

### Solution d'alkoxyphénolate

Selon une variante avantageuse on prépare une solution d'alkoxyphénolate. Cette solution d'alkoxyphénolate est alimentée en l'alkoxyphénol à partir d'une cuve de stockage qui reçoit l'alkoxyphénol recyclé de la boucle de recyclage 1 et l'alkoxyphénol neuf. Cette solution d'alkoxyphénolate est avantageusement introduite dans le réacteur pour la réaction de condensation.

La totalité de l'eau (déminéralisée) et d'une base minérale (par exemple soude) nécessaires à la réaction sont alimentés dans la solution d'alkoxyphénolate.

La température du réacteur de préparation de la solution d'alkoxyphénolate est avantageusement maintenue à la température de la réaction de condensation.

### Boucle de recyclage 1

La boucle de recyclage 1 est la récupération de l'alkoxyphénol n'ayant pas réagi à la réaction de condensation. Ainsi la boucle 1 concerne la séparation du composé alkoxyphénol des composés mandéliques, qui comprend avantageusement les étapes suivantes :
- récupération d'un mélange comprenant le composé mandélique formé sous forme de mandélate et de l'alkoxyphénol n'ayant pas réagi sous forme d'alkoxyphénolate,
- neutralisation de l'alkoxyphénolate en l'alkoxyphénol en maintenant le mandélate correspondant sous forme de base conjuguée (salifiée) ;
- séparation de l'alkoxyphénol du mandélate ;
- introduction du mandélate dans un réacteur d'oxydation ; et
- recyclage de l'alkoxyphénol, n'ayant pas réagi comme réactif, à la réaction de condensation.

Le milieu réactionnel issu de la condensation est avantageusement neutralisé par un acide minéral, et de préférence par l'acide sulfurique, pour transformer l'alkoxyphénolate n'ayant pas réagi en l'alkoxyphénol dans des conditions conservant le mandélate sous forme salifiée.

On peut séparer l'alkoxyphénol n'ayant pas réagi, présent en phase organique, du mandélate formé présent en phase aqueuse.

Selon une variante, on peut extraire l'alkoxyphénol obtenu après neutralisation par extraction par ajout d'un solvant organique qui solubilise l'alkoxyphénol contenu dans la phase aqueuse. On fait référence ici par exemple au procédé décrit pour le gaïacol dans le brevet FR 2 379 501.

Selon un mode de réalisation préféré, l'alkoxyphénol contenu dans la phase aqueuse est extrait par un solvant organique. On fait appel à un solvant organique qui soit inerte par rapport au mandélate. Comme solvants susceptibles d'être utilisés, on peut citer notamment les hydrocarbures aliphatiques, cycloaliphatiques, aromatiques, halogènes ou non ; les alcools ; les éthers, les cétones et les nitriles. On mentionne plus particulièrement comme hydrocarbures aliphatiques, cycloaliphatiques ou aromatiques, l'heptane, le cyclohexane, le méthylcyclohexane, le benzène, le toluène ; comme hydrocarbures aliphatiques, cycloaliphatiques ou aromatiques halogènes, le dichlorométhane, le trichlorométhane, le dichloroéthane, le chlorobenzène, les dichlorobenzènes ; comme alcools, le méthanol, l'éthanol, le propanol, l'isopropanol, le butanol ; comme éthers, le diéthyléther, le düsopropyléther, le méthyltertiobutyléther, comme cétones, l'acétone, la méthyléthylcétone, la méthylisobutylcétone, la düsobutylcétone, la méthylisobutylcétone ; comme nitriles, l'acétonitrile. On peut mettre en oeuvre un mélange desdits solvants. On peut utiliser un solvant de qualité alimentaire.

Le mandélate reste alors en phase aqueuse alors que l'alkoxyphénol passe en phase organique.

L'alkoxyphénol à recycler peut être contaminé par la présence de résidus de solvant organique. La présence de solvant organique dans l'alkoxyphénol conduit à une baisse importante des performances de la réaction de condensation. Il est donc préférable de séparer le solvant d'extraction de l'alkoxyphénol avant sa réintroduction dans la cuve de stockage de l'alkoxyphénol.

Après séparation des phases aqueuse et organique, le mélange alkoxyphénol-phase organique est de préférence distillé pour séparer le solvant organique de l'alkoxyphénol, ainsi que de goudrons généralement présents, afin de recycler le solvant organique. D'autres types de séparation peuvent être envisagés.

La phase aqueuse contenant le mandélate est de préférence traitée pour éliminer le solvant organique résiduel, par exemple par distillation. Le mandélate alimente ensuite la réaction d'oxydation.

Selon une variante, on peut séparer l'alkoxyphénolate n'ayant pas réagi en utilisant des résines échangeuses d'anions basique ou un adsorbant conduisant à la fixation sélective de l'alkoxyphénol et à la récupération d'un flux aqueux comprenant les composés mandéliques sous forme salifiée issus de la réaction de condensation. On peut séparer le flux de l'alkoxyphénol sous forme salifiée (alkoxyphénolate) fixé sur la résine ou l'adsorbant par un traitement de régénération de la résine ou de désorption. De telles méthodes de séparation sont notamment décrites dans les demandes de brevet WO 09/141280 et WO 11/039331.

L'alkoxyphénol n'ayant pas réagi lors de la réaction de condensation est de préférence recyclé à l'alimentation de la solution d'alkoxyphénolate.

### Elimination du dialkoxybenzène (appelé « DAB ») éventuellement présent

Comme il était indiqué plus haut, la réaction de O-alkylation génère également un composé dialkoxybenzène. Dans le procédé de l'invention l'alkoxyphénol obtenu après la réaction de O-alkylation est donc en mélange avec un composé de suralkoxylation (dialkoxylation) du type dialkoxybenzène.

Il a été en particulier découvert par les inventeurs que lorsque le vératrole (1,2-diméthoxybenzène) est formé, notamment lorsque le gaïacol a été préparé à partir de pyrocatéchol, le vératrole s'accumule dans la solution de gaïacol du fait du recyclage mais aussi car il ne réagi pas lors de la réaction de condensation avec l'acide glyoxylique. La concentration en vératrole dans la cuve de stockage du gaïacol (mélange neuf + recyclé) est variable. Comme le vératrole ne réagit pas dans les réactions de condensation selon l'invention, le vératrole a donc tendance à s'accumuler dans la solution de gaïacol et fait donc baisser le titre de gaïacol dans la solution injectée dans le réacteur de condensation. Dans le procédé de l'invention on cherche à limiter le vératrole accumulé dans le gaïacol ou la solution de gaïacolate. Ainsi selon un mode de réalisation préféré de l'invention, on élimine le dialkoxybenzène formé. Selon une variante du procédé de l'invention la concentration massique en vératrole dans la solution comprenant AP avant la réaction de condensation est comprise entre de 0,01 à 25% et de préférence compris entre 0,01 et 10 %, et encore de préférence compris entre 0,01 et 5 %, et est en général d'environ 2%.

L'invention concerne donc un procédé, indépendamment brevetable, de préparation d'au moins un composé alkoxyhydroxybenzaldéhyde à partir d'au moins un composé hydroxyphénol, ledit procédé étant caractérisé en ce qu'il comprend :
(i) - la synthèse d'au moins un alkoxyphénol à partir d'au moins un hydroxyphénol en présence d'au moins un agent O-alkylant ;
(ii) - la récupération d'au moins un alkoxyphénol et d'impuretés dont un composé dialkylé du type dialkoxybenzène (appelé aussi ici « DAB ») ;
(iii) - la séparation du dialkoxybenzène de l'alkoxyphénol ;
(iv) - la condensation de l'alkoxyphénol avec l'acide glyoxylique et l'obtention du composé mandélique correspondant ;
(v) - l'oxydation du composé mandélique en alkoxyhydroxybenzaldéhyde correspondant ; et
(vi) - la récupération de l'alkoxyhydroxybenzaldéhyde éventuellement sous forme solide.

En particulier l'invention consiste en un procédé de préparation d'au moins un alkoxyhydroxybenzaldéhyde choisi parmi la vanilline (4-hydroxy-3-méthoxybenzaldéhyde), l'éthylvanilline (3-éthoxy-4-hydroxybenzaldéhyde), et l'un quelconque de leur mélanges, à partir de pyrocatéchol.

Selon une variante préférée, l'étape (i) est mise en oeuvre en milieu triphasique comme décrit dans l'invention, incluant toutes les variantes et modes de réalisation décrits sans aucune limitation.

Selon un mode de réalisation préféré, l'étape (iii) de séparation du dialkoxybenzène de l'alkoxyphénol comprend en outre la préparation d'une solution aqueuse d'alkoxyphénol sous forme salifiée (phénolate) ; et la séparation du dialkoxybenzène résiduel du mélange aqueux comprenant l'alkoxyphénoate.

Selon une variante préférée, illustrée par exemple selon la figure 4 le procédé comprend les étapes suivantes:
- préparation d'une solution d'alkoxyphénol (AP) par exemple en mélange formé d'un alkoxyphénol neuf avec un alkoxyphénol provenant de recyclage, ladite solution d'alkoxyphénol comprenant également une impureté du type dialkoxybenzène (DAB) ;
- préparation d'une solution d'alkoxyphénolate par ajout d'eau et d'au moins une base à la solution d'alkoxyphénol ;
- séparation du dialkoxybenzène de l'alkoxyphénol ;
- valorisation éventuelle du dialkoxybenzène ;
- condensation de l'alkoxyphénol avec l'acide glyoxylique et l'obtention du composé mandélique correspondant, par exemple salifié (mandélate).

Selon une variante, la séparation du dialkoxybenzène de l'alkoxyphénol est réalisée par distillation ou décantation.

Avantageusement, les composés mandéliques sont obtenus sous forme salifiée. Ainsi le procédé de l'invention comprend une étape de neutralisation du composé mandélique salifié en composé mandélique sous forme acide.

Selon une variante, l'hydroxyphénol est le pyrocatéchol, l'alkoxyphénol est le gaïacol obtenu à l'étape (i) avec un agent méthylant, et le dialkoxybenzène est le vératrole, et le procédé comprend également la séparation à l'étape (iii) du pyrocatéchol du mélange gaïacol et vératrole.

Selon une variante, l'hydroxyphénol est le pyrocatéchol, l'alkoxyphénol est le guéthol obtenu à l'étape (i) avec un agent éthylant, et le dialkoxybenzène est le 1,2-diéthoxybenzène, et le procédé comprend également la séparation à l'étape (iii) du pyrocatéchol du mélange guéthol et du 1,2-diéthoxybenzène.
Avantageusement, une partie de l'alkoxyphénol (alkoxyphénolate) est avantageusement dérivée vers une colonne de distillation destinée à limiter la teneur en dialkoxybenzène dans le gaïacol avant la réaction de condensation. Le dialkoxybenzène est distillé en tête sous forme hétéro-azéotrope dialkoxybenzène-eau. On préfère un température en pied de colonne de distillation comprise entre 90 et 120°C. Ce procédé permet par exemple pour le gaïacol de limiter la concentration massique en vératrole à 0,01 % dans le gaïacol (ou gaïacolate) utilisé pour la réaction de condensation.

L'hétéro-azéotrope dialkoxybenzène-eau est ensuite avantageusement séparé par décantation ou un autre moyen de séparation.

Le pied de colonne (comprenant l'alkoxyphénolate) est de préférence dirigé vers l'alimentation du réacteur de condensation pour faire réagir par condensation l'alkoxyphénol (alkoxyphénolate) et l'acide glyoxylique.

### Oxydation des composés mandéliques en alkoxyhydroxybenzaldéhyde

L'étape d'oxydation permet la transformation des composés mandéliques en les alkoxyhydroxybenzaldéhydes désirés. Le composé para-mandélique répond plus particulièrement à la formule (IV) précitée. En particulier, on peut préparer la para-vanilline (ici appelé vanilline) sous forme de vanillate par oxydation de l'acide para-mandélique correspondant.

L'alkoxyhydroxybenzaldéhyde est obtenu par oxydation du composé mandélique correspondant, de préférence en atmosphère oxydante, comme O₂ ou l'air.

Selon une variante, le milieu réactionnel est un milieu aqueux alcalin, de préférence une base minérale et encore de préférence de l'hydroxyde de sodium ou de potassium, pour former le phénate correspondant, et pour capter le CO₂ libéré, sous forme de carbonate.

La réaction peut être conduite en continu ou en discontinu, par exemple en milieu fortement dilué dans l'eau.

La réaction peut être catalysée. Un catalyseur de cette réaction d'oxydation peut être choisi parmi les catalyseurs comprenant au moins un élément métallique choisi parmi le groupe formé par le cuivre, le nickel, le cobalt, le fer, le manganèse, et l'un quelconque de leurs mélanges. A titre d'exemples de composés minéraux ou organiques du cuivre, on peut citer notamment comme composés du cuivre, le bromure cuivreux et cuivrique ; l'iodure cuivreux ; le chlorure cuivreux et cuivrique ; le carbonate cuivrique basique ; le nitrate cuivreux et cuivrique ; le sulfate cuivreux et cuivrique ; le sulfite cuivreux ; l'oxyde cuivreux et cuivrique ; l'hydroxyde cuivrique ; l'acétate cuivreux et cuivrique ; le trifluorométhylsulfonate cuivrique. Comme exemples spécifiques de dérivés du nickel, on peut citer les halogénures de nickel (II), tels que chlorure, bromure ou iodure de nickel (II) ; le sulfate de nickel (II) ; le carbonate de nickel (II) ; les sels d'acides organiques comprenant de 1 à 18 atomes de carbone tels que notamment acétate, propionate ; les complexes de nickel (II) tels que acétylacétonate de nickel (II), dichloro-bis-(triphénylphosphine) de nickel (II), le dibromo-bis(bipyridine) de nickel (II) ; les complexes de nickel (0) tels que le bis-(cycloocta-1 ,5-diène) de nickel (0), le bis-diphénylphosphinoéthane de nickel (0). Comme exemples de composés à base de cobalt, on peut citer notamment les halogénures de cobalt (II) et (III) tels que chlorure, bromure ou iodure de cobalt (II) chlorure, bromure ou iodure de cobalt (III) ; le sulfate de cobalt (II) et de cobalt (III) ; le carbonate de cobalt (II), le carbonate basique de cobalt (II) ; l'orthophosphate de cobalt (II) ; le nitrate de cobalt (II) ; l'oxyde de cobalt (II) et de cobalt (III) ; l'hydroxyde de cobalt (II) et de cobalt (III) ; les sels d'acides organiques comprenant de 1 à 18 atomes de carbone tels que notamment l'acétate de cobalt (II) et de cobalt (III), le propionate de cobalt (II) ; les complexes de cobalt (II) tels que le chlorure d'hexaminecobalt (II) ou (III), le sulfate d'hexaminecobalt (II) ou (III), le chlorure de pentaminecobalt (III), le chlorure de triéthylènediaminecobalt (III). On peut également faire appel à des système catalytiques à base de fer, généralement sous la forme d'oxydes, d'hydroxydes ou de sels tels que le chlorure, le bromure, l'iodure, le fluorure de fer (II) et de fer (III) ; le sulfate de fer (II) et de fer (III) ; le nitrate de fer (II) et de fer (III) ; l'oxyde de fer (II) et de fer La réaction d'oxydation peut être catalysée par exemple par un système catalytique comprenant deux éléments métalliques choisis parmi le groupe formé par le cuivre, le nickel, le cobalt, le fer, le manganèse, et l'un quelconque de leur mélanges. La présente invention couvre en particulier les réactions décrites selon la demande de brevet WO 08/148760.

Dans un 1^{er} temps, le composé mandélate réagit avec la base (de préférence la soude) pour salifier la fonction phénate du composé mandélate. Puis l'oxydation en milieu oxydant (de préférence à l'air) produit le vanillate, et du CO₂ (piégé sous forme de carbonate) :
L'oxydation du para-mandélique en para-vanilline est quasi complète, avec une très bonne sélectivité.

A l'issue de la réaction d'oxydation, on obtient le précurseur de l'alkoxyhydroxybenzaldéhyde (vanilline et/ou d'éthylvanilline) c'est-à-dire avec un groupe hydroxyle sous forme salifiée (ionique), et diverses impuretés, dont des goudrons.

Dans une étape suivante, on effectue l'acidification de l'alkoxyhydroxybenzaldéhyde (vanilline et/ou d'éthylvanilline) dans le milieu réactionnel à l'aide d'un acide fort, par exemple l'acide sulfurique. Il faut ensuite récupérer le produit noble à savoir l'alkoxyhydroxybenzaldéhyde (vanilline et/ou d'éthylvanilline) en présence de goudrons selon un procédé qui soit à la fois respectueux de l'intégrité des produits à séparer tout en étant économique et facile à mettre en oeuvre dans des dispositifs industriels tout particulièrement dans ceux qui fonctionnent en continu. Pour séparer l'alkoxyhydroxybenzaldéhyde (vanilline et/ou d'éthylvanilline) du mélange brut réactionnel, une méthode connue consiste à effectuer son extraction à l'aide d'un solvant organique.

Avantageusement le procédé comprend :
- la séparation de l'alkoxyhydroxybenzaldéhyde du mélange réactionnel par extraction avec un solvant organique ; et
- la récupération et le recyclage du solvant organique utilisé pour l'extraction.

### « Boucle de recyclage 2 »

La boucle de recyclage 2 couvre la transformation de l'hydroxylate d'alkoxybenzaldéhyde (vanillate et/ou d'éthylvanillate) obtenu après la réaction d'oxydation en alkoxyhydroxybenzaldéhyde correspondant, et la récupération et le recyclage du solvant utilisé pour l'extraction de l'alkoxyhydroxybenzaldéhyde (vanilline et/ou d'éthylvanilline) du mélange issu de la réaction d'oxydation. L'invention couvre en particulier la préparation de vanilline et/ou d'éthylvanilline.

Le milieu réactionnel issu de l'oxydation est acidifiée par un milieu acide, de préférence contenant un acide minéral, et par exemple de l'acide sulfurique, ou l'acide chlorhydrique, pour transformer le sel d'alkoxyhydroxybenzaldéhyde en alkoxyhydroxybenzaldéhyde.

Selon un mode de réalisation préféré, l'alkoxyhydroxybenzaldéhyde contenu dans la phase aqueuse est extrait par un solvant organique qui solubilise l'alkoxyhydroxybenzaldéhyde contenu dans la phase aqueuse. On fait appel à un solvant organique qui soit inerte par rapport à l'alkoxyhydroxybenzaldéhyde. Comme solvants susceptibles d'être utilisés, on peut citer notamment les hydrocarbures aliphatiques, cycloaliphatiques, aromatiques, halogènes ou non ; les alcools ; les éthers, les cétones et les nitriles. On mentionne plus particulièrement comme hydrocarbures aliphatiques, cycloaliphatiques ou aromatiques, l'heptane, le cyclohexane, le méthylcyclohexane, le benzène, le toluène ; comme hydrocarbures aliphatiques, cycloaliphatiques ou aromatiques halogènes, le dichlorométhane, le trichlorométhane, le dichloroéthane, le chlorobenzène, les dichlorobenzènes ; comme alcools, le méthanol, l'éthanol, le propanol, l'isopropanol, le butanol ; comme éthers, le diéthyléther, le düsopropyléther, le méthyltertiobutyléther, comme cétones, l'acétone, la méthyléthylcétone, la méthylisobutylcétone, la düsobutylcétone ; comme nitriles, l'acétonitrile. On peut mettre en oeuvre un mélange desdits solvants.

L'opération d'extraction est effectuée à une température qui dépend de la nature du solvant.

Cette étape d'extraction peut être réalisée en même temps que l'acidification. Cependant on préfère que le milieu réactionnel et le solvant organique soient mélangés ensemble, puis que l'acide soit ajouté.

Pour isoler l'alkoxyhydroxybenzaldéhyde (vanilline et/ou d'éthylvanilline) du solvant d'extraction, on peut réaliser une séparation par recristallisation mais de préférence on effectue une distillation dudit mélange permettant d'obtenir par exemple en tête de distillation le solvant d'extraction (s'il est le composé le plus volatil du mélange), et par exemple en pied de distillation, un « alkoxyhydroxybenzaldéhyde brut" (vanilline et/ou d'éthylvanilline « brutes »), à savoir un mélange comprenant essentiellement l'alkoxyhydroxybenzaldéhyde, associé à des impuretés lourdes dénommées "goudrons" et à de faibles quantités d'impuretés légères. On fait référence pour cette étape en particulier au procédé décrit dans la demande de brevet WO 2010/007161.

Le solvant organique utilisé est avantageusement recyclé. Il peut être réintroduit dans le procédé pour extraire l'alkoxyhydroxybenzaldéhyde de la phase aqueuse lors de l'acidification, et/ou utilisé pour récupérer l'alkoxyhydroxybenzaldéhyde des eaux de lavage.

Eventuellement l'alkoxyhydroxybenzaldéhyde peut être traité pour le conditionner sous forme solide. De préférence l'alkoxyhydroxybenzaldéhyde purifié, de préférence par distillation, suivie d'une cristallisation (par utilisation d'un ou plusieurs solvants ou par technique d'écaillage), par exemple selon les étapes suivantes :
- dissolution de l'alkoxyhydroxybenzaldéhyde dans un mélange eau-alcool (par exemple méthanol ou éthanol) ;
- cristallisation sous vide ;
- essorage en discontinu ;
- séchage de l'alkoxyhydroxybenzaldéhyde à une température de 25°C à une température inférieure à la température de fusion de l'alkoxyhydroxybenzaldéhyde, de préférence sous gaz inerte (azote typiquement).

On peut envisager plusieurs variantes de cristallisation : par exemple cristallisation discontinue (batch) par exemple par refroidissement, ou cristallisation continue, par exemple sous vide.
Le produit résultant peut être éventuellement broyé pour obtenir des grains plus fins.

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

### Exemples

Les pourcentages sont donnés en poids par rapport au poids de la composition totale. Les températures sont données en degré Celsius (°C).

### Exemple 1

On fait réagir en continu en phase vapeur le catéchol avec le méthanol après vaporisation. Le mélange résultant comprend du catéchol, du gaïacol, du vératrole, du méthyl-6-gaïacol, du méthanol n'ayant pas réagi, et des goudrons (composé de forte masse moléculaire par rapport aux autres composés environnants, par exemple masse moléculaire multipliée par 2 ou plus). Selon les conditions opératoires observées les proportions du vératrole, du méthyl-6-gaïacol et des goudrons peuvent être plus ou moins importantes dans le flux liquide condensé sortant de la réaction. Typiquement cette réaction peut être réalisée selon le brevet EP 914 854 B1.

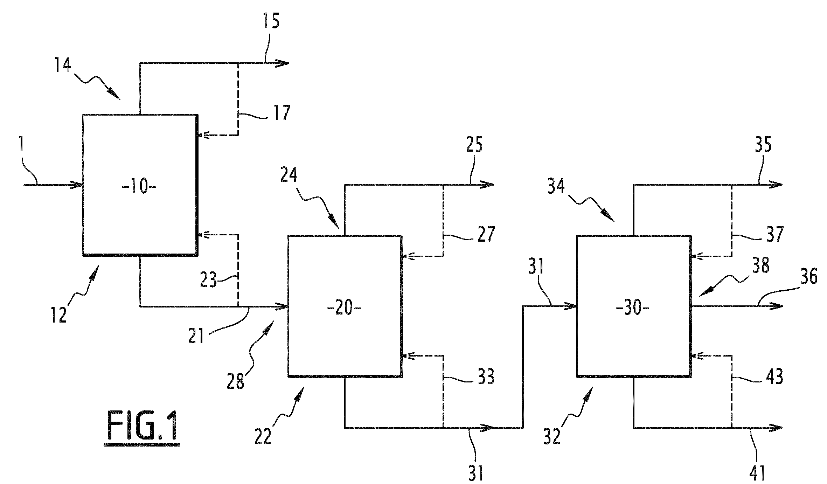

Le mélange résultant précité a été soumis à une distillation continue en utilisant le système de distillation montré sur la figure 1 comprenant une première colonne 10 de distillation (A), une deuxième colonne 20 de distillation (E), et une troisième colonne 30 de distillation (F), raccordées successivement en série.

Le mélange liquide 1 contenant du catéchol, du gaïacol, du vératrole, du méthyl-6-gaïacol, du méthanol n'ayant pas réagi, et des goudrons a été alimenté dans la partie centrale de la première colonne 10 de distillation comprenant dans sa partie basse 12 un bouilleur à film liquide tombant. La température d'évaporation était de 242°C en moyenne. On a fait fonctionner la première colonne 10 de distillation dans les conditions classiques pour séparer selon deux flux ou fractions distinct : le flux ou fraction 15 de tête soutiré de la partie haute 14 de colonne 10 comprenant essentiellement le méthanol, et le flux ou fraction de pied 21 soutiré de la partie basse 12 de colonne 10 comprenant essentiellement les autres composés plus lourds. Le flux ou fraction de tête 15 de la partie haute 14 de colonne 10 a été refroidi par un refroidisseur pour condenser au moins partiellement le flux ou fraction de tête 15. Une partie du flux ou fraction 15 de tête peut être introduite selon un flux ou fraction 17 dans la partie haute 14 de colonne 10. Le flux de sortie 15 de la partie haute 14 de colonne 10 peut être purifié séparément par exemple à nouveau par une distillation mettant en oeuvre une ou plusieurs colonnes de distillation. Avantageusement le méthanol n'ayant pas réagi est recyclé vers les réactifs de la réaction d'O-alkylation, après purification. Dans la partie basse 12, une partie du flux ou fraction de pied 21 soutiré peut être réintroduit partiellement dans la partie basse de la colonne 10 selon un flux ou fraction 23.

On peut ainsi typiquement obtenir un flux ou fraction de pied 21 sortant de la partie basse 12 de cette première colonne 10 de distillation comprenant :
Catéchol (PC) : 400 à 600 kg ;
Gaïacol (GA) :400 à 600 kg ;
Vératrole (VER) : 10 à 50 kg ;
Méthyl-6-gaïacol (MEGA) : 5 à 50 kg ;
Goudrons : 5 à 100 kg.

Le flux de pied 21 acheminé de la partie basse 12 de la première colonne 10 de distillation et ayant la température d'environ 115°C a été alimenté dans une partie centrale 28 d'une deuxième colonne de distillation 20 et un bouilleur à film liquide tombant.

Dans la deuxième colonne 20 de distillation, une fraction de tête 25 comprenant essentiellement du guaïacol et une fraction de pied 31 comprenant essentiellement du catéchol ont été générées à partir du flux liquide 21 alimenté. La température d'évaporation dans le bouilleur était de 155°C en moyenne, et la température dans la partie haute 24 de colonne 20 était d'environ 110°C Dans la partie basse 22, une partie du flux ou fraction de pied 31 soutiré peut être réintroduite dans la partie basse de la colonne 20 selon un flux ou fraction 33. Une partie du flux de tête 25 soutiré peut être introduite selon un flux ou fraction 27 dans la partie haute 24 de la deuxième colonne 20.

La distillation dans la colonne 20 est mise en oeuvre pour sortir de la partie haute 24 de colonne un maximum de gaïacol et en pied un maximum de catéchol, de vératrole et de MEGA-6. Le flux liquide de pied 31 soutiré de la partie basse 22 de la seconde colonne de distillation 20 a été récupéré à un débit moyen d'environ 650 kg/heure. Le flux de tête 25 soutiré de la partie haute 24 de la seconde colonne de distillation 20 a été récupéré à un débit moyen d'environ 570 kg/heure.

Le flux de tête 25 récupéré de la partie haute de 24 de colonne 20 comprend avantageusement :

| | |
|---|---|
| Catéchol (PC) : | environ 0 kg ; |
| Gaïacol (GA) : | 400 à 600 kg ; |
| Vératrole (VER) : | 0 à 5 kg ; |
| Méthyl-6-gaïacol (MEGA) : | 0 à 5 kg ; |
| Goudrons : | environ 0 kg. |

Le flux de pied 31 récupéré de la partie basse de 22 de colonne 20 comprend avantageusement :

| | |
|---|---|
| Catéchol (PC) : | 400 à 600 kg ; |
| Gaïacol (GA) : | 0 à 50 kg ; |
| Vératrole (VER) : | 5 à 50 kg ; |
| Méthyl-6-gaïacol (MEGA) : | 0 à 50 kg ; |
| Goudrons : | 5 à 100 kg. |

Le flux de pied 31 acheminé de la partie basse 22 de la seconde colonne 20 de distillation a été alimenté dans une partie centrale 38 d'une troisième colonne de distillation 30 et un bouilleur à film liquide tombant.

La distillation dans la troisième colonne 30 est mise en oeuvre pour soutirer de la partie haute 34 de colonne un flux de tête 35 comprenant du gaïacol, du vératrole, du MEGA-6, et du catéchol, pour soutirer dans la partie centrale 38 un flux latéral 36 comprenant essentiellement du catéchol, et pour soutirer dans la partie basse 32 un flux de pied 41 comprenant un maximum de goudrons.

Le flux de tête 35 contenant majoritairement le catéchol avait une température d'environ 140°C et a été récupéré à un débit moyen d'environ 95 kg/heure de la partie haute 34 de la troisième colonne de distillation 30.

Dans la partie basse 32 de la colonne 30, une partie du flux ou fraction de pied 41 soutiré peut être réintroduite dans la partie basse 32 selon un flux ou fraction 33. Dans la partie haute 34 de la colonne 30, une partie du flux ou fraction de tête 35 soutiré peut être réintroduite dans la partie haute 34 selon un flux ou fraction 37.

Le flux de pied 41 contenant majoritairement le catéchol concentré avait une température d'environ 210°C et a été récupéré à un débit moyen d'environ 68 kg/heure de la partie basse 32 de la troisième colonne de distillation 30.

Le flux liquide 36 contenant majoritairement le catéchol concentré avait une température d'environ 155°C et a été récupéré à un débit moyen d'environ 485 kg/heure de la partie centrale 38 de la troisième colonne de distillation 30.

Le flux latéral 36 récupéré de la partie centrale 38 de colonne 30 comprend donc avantageusement :

| | |
|---|---|
| Catéchol (PC) : | environ 400 à 600 kg ; |
| Gaïacol (GA) : | 0 à 20 kg ; |
| Vératrole (VER) : | 0 à 50 kg ; |
| Méthyl-6-gaïacol (MEGA) : | 0 à 10 kg ; |
| Goudrons : | environ 0 kg. |

Le flux de tête 35 récupéré de la partie haute 34 de colonne 30 comprend donc avantageusement :

| | |
|---|---|
| Catéchol (PC) : | 0 à 100 kg ; |
| Gaïacol (GA) : | 0 à 50 kg ; |
| Vératrole (VER) : | 0 à 50 kg ; |
| Méthyl-6-gaïacol (MEGA) : | 0 à 50 kg ; |
| Goudrons : | environ 0 kg. |

Le flux de pied 41 récupéré de la partie basse 32 de colonne 30 comprend donc avantageusement :

| | |
|---|---|
| Catéchol (PC) : | 0 à 10 kg ; |
| Gaïacol (GA) : | environ 0 kg ; |
| Vératrole (VER) : | environ 0 kg ; |
| Méthyl-6-gaïacol (MEGA) : | environ 0 kg ; |
| Goudrons : | 5 à 100 kg. |

Les colonnes de distillation 20 et 30 permettent avantageusement de séparer d'une part un flux de tête 25 en sortie haute de deuxième colonne 20, sous forme de vapeur éventuellement condensée, contenant majoritairement le gaïacol et un flux latéral 36 en sorite de troisième colonne de distillation 30, sous forme de vapeur éventuellement condensée, contenant majoritairement le catéchol.

Le vératrole et l'impureté méthyl-6-gaïacol peuvent donc être séparés du flux contenant le gaïacol de manière satisfaisante à son utilisation ultérieure pour la préparation de composés aromatiques utilisés notamment dans l'industrie alimentaire, comme la vanilline. Le vératrole et l'impureté méthyl-6-gaïacol peuvent aussi être séparés du flux contenant le catéchol concentré de manière suffisante pour prévoir un recyclage industriel du catéchol. Le procédé selon la présente invention permet le recyclage du gaïacol, ainsi que le recyclage du catéchol, et présente un avantage économique industriel important notamment de par l'efficacité de la séparation, que ce soit en termes de pureté et de rendement des produits distillés, et de rentabilité économique.

### Exemple 2

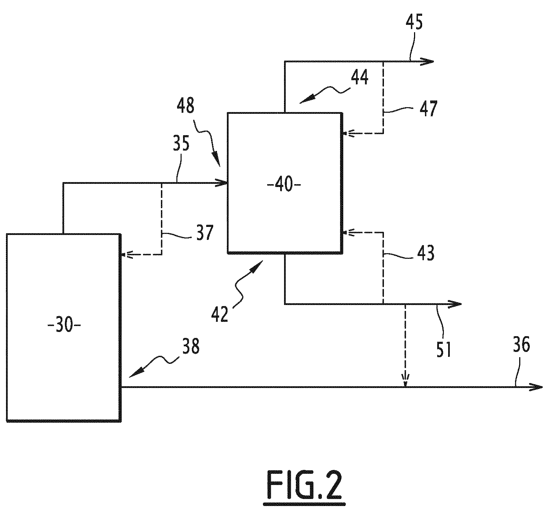

On peut introduire le flux ou fraction de tête 35 soutiré de la troisième colonne 30 selon l'exemple 1 dans une quatrième colonne de distillation 40 (F2), comme illustré par la figure 2.

Dans la quatrième colonne 40 de distillation, un flux ou fraction de pied 51 comprenant essentiellement du catéchol, et un flux ou fraction de tête 45 contenant essentiellement un mélange de gaïacol, de vératrole, et de MEGA, ont été générées à partir du flux de tête 35 alimenté.

La distillation dans la quatrième colonne 40 est mise en oeuvre pour soutirer de la partie haute 44 de colonne un flux de tête 45 comprenant du gaïacol, du vératrole, et majoritairement MEGA, et pour soutirer dans la partie basse 42 un flux de pied 51 comprenant du catéchol, du vératrole et une faible proportion de MEGA.

Dans la partie basse 42 de la colonne 40, une partie du flux ou fraction de pied 51 soutiré peut être réintroduite dans la partie basse 42 selon un flux ou fraction 43. Dans la partie haute 44 de la colonne 40, une partie du flux ou fraction de tête 45 soutiré peut être réintroduite dans la partie haute 44 selon un flux ou fraction 47 (non représenté).

Le flux de tête 45 avait une température d'environ 115°C et a été récupéré à un débit moyen d'environ 22 kg/heure de la partie haute 24 de la seconde colonne de distillation 20.

Le flux de pied 51 avait une température d'environ 145°C et a été récupéré à un débit moyen d'environ 69 kg/heure de la partie haute 44 de la quatrième colonne de distillation 40.

Le flux de pied 51 récupéré de la partie basse 42 de colonne 40 comprend donc avantageusement :

| | |
|---|---|
| Catéchol (PC) : | 40 à 100 kg ; |
| Gaïacol (GA) : | de 0 à 5 kg ; |
| Vératrole (VER) : | de 0 à 10 kg ; |
| Méthyl-6-gaïacol (MEGA) : | de 0 à 5 kg ; |
| Goudrons : | environ 0 kg. |

Le flux 51 peut être avantageusement ajouté partiellement ou totalement au flux de soutirage latéral 36 de la colonne 30 avant le recyclage en amont de la réaction de O-alkylation.

Le flux de tête 45 récupéré de la partie haute 44 de colonne 40 comprend avantageusement :

| | |
|---|---|
| Catéchol (PC) : | environ 0 kg ; |
| Gaïacol (GA) : | de 0 à 20 kg ; |
| Vératrole (VER) : | de 0 à 10 kg ; |
| Méthyl-6-gaïacol (MEGA) : | de 5 à 50 kg ; |
| Goudrons : | environ 0 kg. |

### Exemple 3

On peut utiliser le flux ou fraction (25) comprenant le gaïacol pour préparer la vanilline de haute pureté au regard de l'impureté MEGA. Pour la synthèse de vanilline à partir de gaïacol, on peut procéder à des étapes de condensation et d'oxydation. Ces étapes sont illustrées ci-dessous dans le cadre de la synthèse de gaïacol obtenu en milieu triphasique (G/L/L), mais sont directement transposables à la préparation de vanilline à partir de gaïacol obtenu en phase vapeur.

### Condensation

Dans un premier réacteur en inox 316L de 5 L muni d'une double enveloppe, d'une agitation mécanique, d'une électrode de pH, d'un système réfrigérant et d'une arrivée de gaz inerte, on charge en continu :
- 64,8 kg/h d'eau déminéralisée
- 9,3 kg/h (116 mol/h) d'une solution aqueuse de soude à 50% en poids.
- 10,1 kg/h (81,5 mol/h) de gaïacol (neuf et recyclé dont on a enlevé tout ou partie du vératrole).

Ce mélange réactionnel est maintenu à la température de 35°C. Cette préparante est ensuite alimentée dans le premier réacteur d'un système de 3 réacteurs en cascade avec une solution aqueuse d'acide glyoxylique à 50% en poids (6,04 kg/h soit 40,8 mol/h).

Les 3 réacteurs parfaitement agités sont en inox 316L et ont chacun un volume de 80 L ; ils opèrent à 35°C.

Le temps de séjour global est de 2 heures.

En sortie du dernier réacteur, on prélève un échantillon de ce milieu réactionnel et on dose par chromatographie en phase liquide les composés présents dans le mélange.

Les résultats obtenus sont les suivants :
- conversion du gaïacol (GA) : TT = 47%
- acide 4-hydroxy-3-méthoxy mandélique (APM) :
   RT(APM)/GA=86%
- acide 2-hydroxy-3-méthoxy mandélique :
   RT(AOM)/GA=5%
- acide 2-hydroxy-3-méthoxy 1,5-dimandélique :
   RT(ADM/GA)=9%

Le milieu réactionnel est envoyé vers la boucle de recylage 1 pour séparer le gaïacol en excès des composés mandéliques.

### Oxydation

Le réacteur d'oxydation en inox 316L équipé d'une agitation autoaspirante de type à cavitation ("cavitator") ou de type Rushton et d'une double enveloppe permettant un refroidissement efficace est alimenté en continu par :
- Le mélange des co-catalyseurs et de la solution aqueuse de mandéliques issue de la boucle 1 soit :
   ∘ 77 kg/h de milieu réactionnel issu de la réaction de condensation dans lequel le gaïacol en excès est éliminé (puis recyclé) dans la boucle de recylage 1. Ce mélange contient de l'ordre de 7,2 kg/h d'acide 4-hydroxy-3-méthoxy mandélique, 0,5 kg/h d'acide 2-hydroxy-3-méthoxy mandélique et 0,9 kg/h d'acide 2-hydroxy-3-méthoxy 1,5-dimandélique.
   ∘ 100 g/h d'une solution aqueuse de CoCl₂ et de CuSO₄ en une quantité exprimée en pourcentage molaire de métal par rapport à la somme molaire des acides mandéliques : 0,04% chacun.
- la quantité adéquate d'une solution aqueuse de soude à 50% en poids correspondant au moins à la quantité requise par la stoechiométrie de la réaction d'oxydation (à discuter ensemble).
- La quantité d'oxygène à pression atmosphérique suffisante pour avoir une conversion quasi complète des acides mandéliques. L'oxydant peut être de l'oxygène à pression atmosphérique ou de l'air sous pression.

Cette réaction s'effectue à 80°C. En sortie de réacteur, on prélève un échantillon de ce milieu réactionnel et on dose les composés présents dans le mélange par chromatographie en phase liquide.

Les résultats obtenus sont les suivants :
- conversion de l'acide 4-hydroxy-3-méthoxy mandélique : TT > 99,5%
- Rendement en vanilline VA :
   RT(VA)/APM=98%

Le milieu réactionnel est envoyé vers la boucle d'extraction du vanillate de sodium de la phase aqueuse (dite boucle de recyclage 2).

## Revendications

1. Procédé de préparation d'au moins un alkoxyhydroxybenzaldéhyde (« AHBA ») à partir d'au moins un hydroxyphénol (« HP »), ledit procédé étant **caractérisé en ce qu'**il comprend la formation d'au moins un alkoxyphénol (« AP ») et d'alkyl-alkoxyphénol (« AAP ») la séparation (S) de AP de AAP, et la préparation dudit AHBA par ajout d'une fonction aldéhyde sur le composé AP, ladite séparation (S) étant mise en oeuvre avant l'obtention de AHBA, la séparation étant réalisée par la mise en oeuvre d'une ou plusieurs étapes de distillation de AP et de AAP en présence de HP.

2. Procédé de préparation selon la revendication précédente, **caractérisé en ce que** l'alkoxyhydroxybenzaldéhyde (AHBA) est la vanilline (VA), l'éthylvanilline (EVA), ou l'un quelconque de leurs mélanges.

3. Procédé de préparation selon la revendication 2, **caractérisé en ce qu'**il comprend la préparation de VA et/ou EVA, sensiblement exempte de 5-méthylvanilline (« MEVA ») et de 3-éthoxy-5-éthyl-4-hydroxy-benzaldéhyde (« EEVA »), à partir de gaïacol (GA) et/ou de guéthol (GE), ledit procédé comprenant l'obtention d'un mélange (M) comprenant GA et/ou GE en présence de l'impureté 6-méthylgaïacol (« MEGA ») et/ou 6-éthylguéthol (2-éthoxy-6-éthyl-phénol dit « EGE »), ledit procédé comprenant la séparation (S) de GA et/ou GE de MEGA et/ou EGE, ladite séparation (S) étant mise en oeuvre avant l'obtention de VA et/ou EVA.

4. Procédé selon l'une quelconque des revendications précédentes, **caractérisé en ce que** le composé AHBA est préparé par ajout d'une fonction aldéhyde au composé AP par condensation avec l'acide glyoxylique puis oxydation du composé résultant, et **caractérisé en ce que** la séparation (S) est réalisée avant l'oxydation en composé AHBA.

5. Procédé selon la revendication 4, **caractérisé en ce que** la séparation est réalisée avant la réaction de AP avec l'acide glyoxylique.

6. Procédé selon l'une quelconque des revendications précédentes, **caractérisé en ce qu'**il comprend l'introduction d'un flux ou fraction (E1) d'un mélange M comprenant du gaïacol (GA) et/ou guéthol (GE), désigné(s) par « G », et 6-méthylgaïacol (MEGA) et/ou 6-éthylguéthol (EGE), désigné(s) par « AG », en présence de pyrocathécol (PC) dans une colonne de distillation (E) et la récupération de G essentiellement en tête de colonne de distillation dans un flux ou fraction (E2) et de PC et AG essentiellement en pied de colonne de distillation dans un flux ou fraction (E3).

7. Procédé selon la revendication 6 **caractérisé en ce que** ledit mélange M comprend
également un dialkoxybenzaldéhyde (DAB), DAB étant en particulier le vératrole (VER), et **en ce que** DAB est récupéré essentiellement en pied de colonne de distillation dans le flux ou fraction (E3).

8. Procédé, selon la revendication 6 ou 7, **caractérisé en ce que** PC représente de 25 à 75 %, et de préférence de 35 à 65%, et encore de préférence de 40 à 45 %, en poids du poids total du flux ou fraction (E1).

9. Procédé, selon l'une quelconque des revendications 6 à 8, **caractérisé en ce que** G représente de 25 à 75 %, et de préférence de 35 à 65%, et encore de préférence de 45 à 50 %, en poids du poids total du flux ou fraction (E1).

10. Procédé, selon l'une quelconque des revendications 6 à 9 **caractérisé en ce que** G représente plus de 90 %, et de préférence plus de 95 %, et encore de préférence plus de 99%, en poids du poids total du flux ou fraction (E2), et de préférence **caractérisé en ce que** AG représente moins de 1 %, et de préférence moins de 0,5 % et encore de préférence moins de 0,2 %, en poids par rapport au poids total du flux ou fraction (E2).

11. Procédé, selon l'une quelconque des revendications 6 à 10, **caractérisé en ce que** flux ou fraction (E2) comprend de 90 à 100%, et de préférence de 95 à 100 %, et encore de préférence de 99 à 100 %, de G, de 0 à 1% et de préférence moins de 0,5 % et encore de préférence moins de 0,2 %, de AG ; et de 0 à 1% et de préférence moins de 0,5 % et encore de préférence moins de 0,2 %, de VER, les pourcentages étant exprimés en poids par rapport au poids total du flux ou fraction (E2).

12. Procédé, selon l'une quelconque des revendications 6 à 11, **caractérisé en ce que** PC représente plus de 60 %, et de préférence plus de 70 %, et encore de préférence plus de 75%, en poids du poids total du flux ou fraction (E3), et de préférence **caractérisé en ce que** AG représente moins de 8 %, et de préférence moins de 5 % et encore de préférence moins de 3 %, en poids par rapport au poids total du flux ou fraction (E3).

13. Procédé, selon l'une quelconque des revendications 6 à 12, **caractérisé en ce que** le flux ou fraction (E3) comprend de 60 à 100%, et de préférence de 70 à 100 %, et encore de préférence de 75 à 100 %, de PC, de 0 à 8% et de préférence moins de 5 % et encore de préférence moins de 3 %, de AG ; et de 0 à 10% et de préférence moins de 8 % et encore de préférence moins de 5 %, de VER, les pourcentages étant exprimés en poids par rapport au poids total du flux ou fraction (E3).

14. Procédé, selon l'une quelconque des revendications 6 à 12, **caractérisé en ce qu'**il comprend une distillation supplémentaire dans une colonne (F) du flux ou fraction (E3) issu du pied de colonne de distillation (E) en réalisant un soutirage latéral d'un flux ou fraction (F6) de la colonne (F) pour soutirer un flux ou fraction enrichie en PC.

## Patentansprüche

1. Verfahren zur Herstellung mindestens eines Alkoxyhydroxybenzaldehyds ("AHBA") aus mindestens einem Hydroxyphenol ("HP"), wobei das Verfahren **dadurch gekennzeichnet ist, dass** es die Bildung mindestens eines Alkoxyphenols ("AP") und von Alkyl-Alkoxyphenol ("AAP"), die Trennung (S) des AP von dem AAP und die Herstellung des AHBA durch Zugabe einer Aldehydfunktion zur Verbindung AP umfasst, wobei die Trennung (S) vor dem Erhalt von AHBA durchgeführt wird, wobei die Trennung ausgeführt wird, indem ein oder mehrere Destillationsschritte von AP und von AAP in Gegenwart von HP durchgeführt werden.

2. Verfahren zur Herstellung nach dem vorhergehenden Anspruch, **dadurch gekennzeichnet, dass** es sich bei dem Alkoxy-hydroxybenzaldehyd (AHBA) um Vanillin (VA), Ethylvanillin (EVA) oder um beliebige Gemische davon handelt.

3. Verfahren zur Herstellung nach Anspruch 2, **dadurch gekennzeichnet, dass** es die Herstellung von VA und/oder EVA, das weitgehend frei von 5-Methylvanillin ("MEVA") und von 3-Ethoxy-5-ethyl-4-hydroxybenzaldehyd ("EEVA") ist, aus Guajacol (GA) und/oder Guethol (GE) umfasst, wobei das Verfahren den Erhalt eines Gemischs (M) umfasst, das GA und/oder GE in Gegenwart der Verunreinigung 6-Methylguajacol ("MEGA") und/oder 6-Ethylguethol (2-Ethoxy-6-ethyl-phenol, genannt "EGE") umfasst, wobei das Verfahren die Trennung (S) von GA und/oder GE von MEGA und/oder EGE umfasst, wobei die Trennung (S) vor dem Erhalt von VA und/oder EVA durchgeführt wird.

4. Verfahren nach einem der vorhergehenden Ansprüche, **dadurch gekennzeichnet, dass** die Verbindung AHBA durch Zugabe einer Aldehydfunktion zur Verbindung AP durch Kondensation mit Glyoxylsäure, dann Oxidation der resultierenden Verbindung hergestellt wird, und **dadurch gekennzeichnet, dass** die Trennung (S) vor der Oxidation zur Verbindung AHBA ausgeführt wird.

5. Verfahren nach Anspruch 4, **dadurch gekennzeichnet, dass** die Trennung vor der Umsetzung von AP mit Glyoxylsäure ausgeführt wird.

6. Verfahren nach einem der vorhergehenden Ansprüche, **dadurch gekennzeichnet, dass** es das Einführen eines Stroms oder einer Fraktion (E1) aus einem Gemisch M, das Guajacol (GA) und/oder Guethol (GE), bezeichnet mit "G", und 6-Methylguajacol (MEGA) und/oder 6-Ethylguethol (EGE), bezeichnet mit "AG", in Gegenwart von Pyrocathecol (PC) in eine Destillationskolonne (E) und das Rückgewinnen von G im Wesentlichen am Kopf der Destillationskolonne in einem Strom oder einer Fraktion (E2) und von PC und AG im Wesentlichen aus dem Sumpf der Destillationskolonne in einem Strom oder einer Fraktion (E3) umfasst.

7. Verfahren nach Anspruch 6, **dadurch gekennzeichnet, dass** das Gemisch M auch ein Dialkoxybenzaldehyd (DAB) umfasst, wobei es sich bei DAB insbesondere um Veratrol (VER) handelt, und dadurch, dass DAB im Wesentlichen aus dem Sumpf der Destillationskolonne in dem Strom oder der Fraktion (E3) rückgewonnen wird.

8. Verfahren nach Anspruch 6 oder 7, **dadurch gekennzeichnet, dass** PC 25 bis 75 Gew.-% und bevorzugt 35 bis 65 Gew.-% und noch stärker bevorzugt 40 bis 45 Gew.-% des Gesamtgewichts des Stroms oder der Fraktion (E1) ausmacht.

9. Verfahren nach einem der Ansprüche 6 bis 8, **dadurch gekennzeichnet, dass** G 25 bis 75 Gew.-% und bevorzugt 35 bis 65 Gew.-% und noch stärker bevorzugt 45 bis 50 Gew.-% des Gesamtgewichts des Stroms oder der Fraktion (E1) ausmacht.

10. Verfahren nach einem der Ansprüche 6 bis 9, **dadurch gekennzeichnet, dass** G mehr als 90 Gew.-% und bevorzugt mehr als 95 Gew.-% und noch stärker bevorzugt mehr als 99 Gew.-% des Gesamtgewichts des Stroms oder der Fraktion (E2) ausmacht, und bevorzugt **dadurch gekennzeichnet, dass** AG weniger als 1 Gew.-% und bevorzugt weniger als 0,5 Gew.-% und noch stärker bevorzugt weniger als 0,2 Gew.-%, bezogen auf das Gesamtgewicht des Stroms oder der Fraktion (E2), ausmacht.

11. Verfahren nach einem der Ansprüche 6 bis 10, **dadurch gekennzeichnet, dass** der Strom oder die Fraktion (E2) 90 bis 100 % und bevorzugt 95 bis 100 % und noch stärker bevorzugt 99 bis 100 % an G, 0 bis 1 % und bevorzugt weniger als 0,5 % und noch stärker bevorzugt weniger als 0,2 % an AG; und 0 bis 1 % und bevorzugt weniger als 0,5 % und noch stärker bevorzugt weniger als 0,2 % an VER umfassen, wobei die Prozentanteile in Gewichtsprozent bezogen auf das Gesamtgewicht des Stroms oder der Fraktion (E2) ausgedrückt werden.

12. Verfahren nach einem der Ansprüche 6 bis 11, **dadurch gekennzeichnet, dass** PC mehr als 60 Gew.-% und bevorzugt mehr als 70 Gew.-% und noch stärker bevorzugt mehr als 75 Gew.-% des Gesamtgewichts des Stroms oder der Fraktion (E3) ausmacht, und bevorzugt **dadurch gekennzeichnet, dass** AG weniger als 8 Gew.-% und bevorzugt weniger als 5 Gew.-% und noch stärker bevorzugt weniger als 3 Gew.-%, bezogen auf das Gesamtgewicht des Stroms oder der Fraktion (E3), ausmacht.

13. Verfahren nach einem der Ansprüche 6 bis 12, **dadurch gekennzeichnet, dass** der Strom oder die Fraktion (E3) 60 bis 100 % und bevorzugt 70 bis 100 % und noch stärker bevorzugt 75 bis 100 % an PC, 0 bis 8 % und bevorzugt weniger als 5 % und noch stärker bevorzugt weniger als 3 % an AG; und 0 bis 10 % und bevorzugt weniger als 8 % und noch stärker bevorzugt weniger als 5 % an VER umfassen, wobei die Prozentanteile in Gewichtsprozent bezogen auf das Gesamtgewicht des Stroms oder der Fraktion (E3) ausgedrückt werden.

14. Verfahren nach einem der Ansprüche 6 bis 12, **dadurch gekennzeichnet, dass** es eine zusätzliche Destillation des Stroms oder der Fraktion (E3), die aus dem Sumpf der Destillationskolonne (E) stammen, in einer Kolonne (F) umfasst, indem ein Seitenabzug eines Stroms oder einer Fraktion (F6) aus der Kolonne (F) ausgeführt wird, um einen Strom oder eine Fraktion abzuziehen, die mit PC angereichert ist.

## Claims

1. Process for producing at least one alkoxyhydroxybenzaldehyde ("AHBA") from at least one hydroxyphenol ("HP"), said process being **characterized in that** it comprises the formation of at least one alkoxyphenol ("AP") and of alkylalkoxyphenol ("AAP"), the separation (S) of AP from AAP, and the production of said AHBA by addition of an aldehyde function to the AP compound, said separation (S) being carried out prior to obtaining AHBA, the separation being carried out by implementing one or more steps of distillation of AP and of AAP in the presence of HP.

2. Production process according to the preceding claim, **characterized in that** the alkoxyhydroxybenzaldehyde (AHBA) is vanillin (VA), ethylvanillin (EVA), or any mixture thereof.

3. Production process according to Claim 2, **characterized in that** it comprises the production of VA and/or EVA, substantially free of 5-methylvanillin ("MEVA") and of 3-ethoxy-5-ethyl-4-hydroxybenzaldehyde ("EEVA"), from guaiacol (GA) and/or from guaethol (GE), said process comprising the obtaining of a mixture (M) comprising GA and/or GE in the presence of the impurity 6-methylguaiacol ("MEGA") and/or 6-ethylguaethol (2-ethoxy-6-ethylphenol termed "EGE"), said process comprising the separation (S) of GA and/or GE from MEGA and/or EGE, said separation (S) being carried out prior to obtaining VA and/or EVA.

4. Process according to any one of the preceding claims, **characterized in that** the AHBA compound is produced by addition of an aldehyde function to the AP compound by condensation with glyoxylic acid, then oxidation of the resulting compound, and **characterized in that** the separation (S) is carried out before the oxidation to give the AHBA compound.

5. Process according to Claim 4, **characterized in that** the separation is carried out before the reaction of AP with glyoxylic acid.

6. Process according to any one of the preceding claims, **characterized in that** it comprises the introduction of a stream or fraction (E1) of a mixture M comprising guaiacol (GA) and/or guaethol (GE), denoted "G", and 6-methylguaiacol (MEGA) and/or 6-ethylguaethol (EGE), denoted "AG", in the presence of pyrocatechol (PC) in a distillation column (E), and the recovery of G essentially at the top of the distillation column in a stream or fraction (E2) and of PC and AG essentially at the bottom of the distillation column in a stream or fraction (E3).

7. Process according to Claim 6, **characterized in that** said mixture M also comprises a dialkoxybenzaldehyde (DAB), DAB being in particular veratrole (VER), and **in that** DAB is recovered essentially at the bottom of the distillation column in the stream or fraction (E3).

8. Process according to Claim 6 or 7, **characterized in that** PC represents from 25% to 75%, and preferably from 35% to 65%, and more preferably from 40% to 45%, by weight of the total weight of the stream or fraction (E1).

9. Process according to any one of Claims 6 to 8, **characterized in that** G represents from 25% to 75%, and preferably from 35% to 65%, and more preferably from 45% to 50%, by weight of the total weight of the stream or fraction (E1).

10. Process according to any one of Claims 6 to 9, **characterized in that** G represents more than 90%, and preferably more than 95%, and more preferably more than 99%, by weight of the total weight of the stream or fraction (E2), and is preferably **characterized in that** AG represents less than 1%, and preferably less than 0.5%, and more preferably less than 0.2%, by weight relative to the total weight of the stream or fraction (E2).

11. Process according to any one of Claims 6 to 10, **characterized in that** the stream or fraction (E2) comprises from 90% to 100%, and preferably from 95% to 100%, and more preferably from 99% to 100% of G, from 0 to 1%, and preferably less than 0.5%, and more preferably less than 0.2% of AG, and from 0 to 1%, and preferably less than 0.5%, and more preferably less than 0.2% of VER, the percentages being expressed by weight relative to the total weight of the stream or fraction (E2).

12. Process according to any one of Claims 6 to 11, **characterized in that** PC represents more than 60%, and preferably more than 70%, and more preferably more than 75%, by weight of the total weight of the stream or fraction (E3), and preferably **characterized in that** AG represents less than 8%, and preferably less than 5%, and more preferably less than 3%, by weight relative to the total weight of the stream or fraction (E3).

13. Process according to any one of Claims 6 to 12, **characterized in that** the stream or fraction (E3) comprises from 60% to 100%, and preferably from 70% to 100%, and more preferably from 75% to 100% of PC, from 0 to 8%, and preferably less than 5%, and more preferably less than 3% of AG, and from 0 to 10%, and preferably less than 8%, and more preferably less than 5% of VER, the percentages being expressed by weight relative to the total weight of the stream or fraction (E3).

14. Process according to any one of Claims 6 to 12, **characterized in that** it comprises an additional distillation, in a column (F), of the stream or fraction (E3) from the bottom of the distillation column (E) by drawing off a stream or fraction (F6) at the side of the column (F) so as to draw off a stream or fraction enriched with PC.
